# EUROPEAN PATENT APPLICATION

(11) **EP 3 560 358 A1**
(43) Date of publication of application: **30.10.2019**
(21) Application number: 17882772.1
(22) Date of filing: 05.12.2017
(51) Int. Cl.: A24F 47/00

(54) **LIQUID INJECTION PROTECTION DEVICE, ATOMIZER AND ELECTRONIC CIGARETTE**

(30) Priority: 21.12.2016 CN 201621406359 U; 01.08.2017 CN 201710647438
(71) Applicant: Changzhou Patent Electronic Technology Co., Ltd, Jiangsu Province 213001 (CN)
(72) Inventor: QIU, Weihua, Xinbei District, Changzhou City Jiangsu Province 213001 (CN); WANG, Wenbang, Xinbei District, Changzhou City Jiangsu Province 213001 (CN)
(74) Representative: Haseltine Lake Kempner LLP
(86) International application number: PCT/CN2017/114549
(87) International publication number: WO 2018/113511

(57) **Abstract**

Provided is a liquid injection protection device, which comprises a cover body (50), a connection assembly (40) and a first locking assembly, wherein a liquid injection port (402) is arranged on the connection assembly (40), the cover body (50) movably covers the liquid injection port (402) relative to the connection assembly (40), the first locking assembly comprises a lock hole arranged on the cover body (50) and a locking member capable of penetrating the lock hole to be detachably connected to the connection assembly (40), and when one end of the locking member penetrates the lock hole to be connected to the connection assembly (40), a movement stroke of the cover body (50) is limited by the locking member, so as to prevent the movement of the cover body to open the liquid injection port (402). In the liquid injection protection device, the liquid injection port (402) can be opened only by firstly releasing the locking member, and then moving the cover body (50). Since a complex operation is required to open the liquid injection port (402), the occurrence of the situation is avoided that a child easily opens the liquid injection port (402), then makes contact with, even accidentally ingests, cigarette liquid, having a protective function for children.

## Description

### TECHNICAL FIELD

The present invention relates to technical field of smoking simulation, and more particularly, relates to a liquid injection protection device, an atomizer and an electronic cigarette.

### BACKGROUND

An atomizer of a prior electronic cigarette includes a housing and a cover body, the housing is provided with a liquid storage chamber therein, the cover body covers an opening end of the liquid storage chamber to prevent leakage of the e-cigarette liquid from the liquid storage chamber. In general, in order to facilitate to perform an e-cigarette liquid injection, the cover body is required to be detachably connected to the housing. However, when the children get this type of electronic cigarette, the cover body may be opened by an accident, and the children may be exposed to, and even ingest, the e-cigarette liquid.

### SUMMARY

Accordingly, it is necessary to provide a liquid injection protection device that is childproof and easy for liquid injection.

It is further necessary to provide an atomizer having the liquid injection protection device.

It is also necessary to provide an electronic cigarette having the atomizer.

The technical solution adopted by the present invention to solve the problem is as follows.

A liquid injection protection device includes a cover body, a connection assembly and a first locking mechanism. The connection assembly is provided with a liquid injection hole. The cover body covers the liquid injection hole and is movable relative to the connection assembly. The first locking mechanism includes a locking hole provided in the cover body and a locking member capable of extending through the locking hole to be detachably connected to the connection assembly. When one end of the locking member extends through the locking hole to be connected to the connection assembly, a movement of the cover body is restricted by the locking member, so as to prevent the movement of the cover body to open the liquid injection hole.

Further, an outer diameter of the portion of the locking member inserted in the locking hole is equal to an inner diameter of the locking hole, and/or an outer wall of the locking member is provided with a resisting edge. When the one end of the locking member extends through the locking hole to be connected to the connection assembly, the cover body is restricted between the resisting edge and the connection assembly.

Further, a sidewall of the locking hole is provided with a restricting protrusion, an external wall of the locking member is provided with a restricting recess, or, the sidewall of the locking hole is provided with a restricting recess, the external wall of the locking member is provided with a restricting protrusion. The restricting protrusion is movably embedded in the restricting recess for preventing a detachment of the locking member from the cover body.

Further, the liquid injection protection device includes a movable ring. The movable ring is rotatably sleeved on an outer side of the connection assembly for preventing the connection assembly from being dismantled.

Further, the locking member is a mouthpiece.

Further, the connection assembly includes a connection base. The cover body is detachably connected to the connection base, or, the cover body is movably arranged on the connection base.

Further, one end of the cover body is hinged to the connection base, allowing the cover body to be flipped relative to the connection base.

Further, a sealing member is protruded from a lower surface of the cover body corresponding to the liquid injection hole. When the cover body covers the connection base, the sealing member is latched into the liquid injection hole.

Further, the cover body is rotatably arranged on the connection base. A rotation center of the cover body is staggered from a central axis of the connection base.

Further, the cover body is slidably arranged on the connection base. The connection base is provided with a sliding rail, and the cover body is provided with a sliding block matching the sliding rail, or, the connection base is provided with a sliding block, and the cover body is provided with a sliding rail matching the sliding block.

Further, the liquid injection protection device includes a second locking mechanism. The second locking mechanism includes a first connecting portion arranged on the connection assembly and a second connecting portion arranged on the cover body. After a connection between the locking member and the connection assembly is removed, the cover body is further required to be moved to remove an engagement between the first connecting portion and the second connecting portion so as to separate the first connecting portion from the second connecting portion, and then the liquid injection hole is able to be opened.

Further, the connection assembly is provided with a sliding groove. The cover body is provided with a rotating shaft. When the cover body is moved, the rotating shaft slides along the sliding groove. When the cover body is moved to a position permitting the first connecting portion to separate from the second connecting portion, the cover body is capable of rotating around the rotating shaft relative to the connection assembly to open the liquid injection hole.

Further, the connection assembly includes a connection base and a gland covering the connection base. The liquid injection hole is provided in the connection base. The gland is provided with a cutout with an opening at one end. The cutout is formed by recessing from one side edge of the gland. The liquid injection hole is received in and exposed from the cutout.

Further, the cutout includes a connecting wall located at a side of the liquid injection hole and two sidewalls respectively connected to opposite ends of the connecting wall and extending in the same direction. The surface of the connecting wall adjacent to the connection base is provided with a recess. The recess is recessed from the connecting wall along a direction away from the cutout. The sliding groove is provided at one end of each sidewall connecting with the connecting wall. The sliding groove is recessed from the surface of the sidewall adjacent to the connection base. The sliding grooves provided at the two surfaces of the two sidewalls are disposed oppositely to each other. The sliding grooves are in communication with the cutout and the recess.

Further, the cover body includes a movable member. The rotating shaft is arranged on opposite sides of one end of the movable member. A covering portion is formed by extending outwardly from opposite sides of the other end of the movable member opposite to the rotating shaft. The gland is provided with a locking groove at a bottom of one end of the gland away from the connecting wall. The locking groove is in communication with the cutout. The covering portion serves as the second connecting portion. The locking groove serves as the first connecting portion. When the movable member is moved to cause the covering portion to engage into the locking groove, one end of the movable member adjacent to the rotating shaft is restricted in the recess. When the movable member is moved to cause the covering portion to disengage from the locking groove, the one end of the movable member adjacent to the rotating shaft is moved out of the recess and the movable member is able to be flipped.

Further, the connection assembly includes a lining member and a stopping member. The liquid injection hole is provided in the lining member. The lining member is further provided with an embedding groove. The stopping member is embedded in the embedding groove. The sliding groove is provided at one end of the stopping member along a length direction of the stopping member. The cover body includes a clamping member. The rotating shaft is arranged on one end of the clamping member.

Further, the connection assembly further includes a snapping member. The snapping member is arranged in the embedding groove and located under one end of the stopping member away from the sliding groove. The one end of the stopping member away from the sliding groove is provided with an insertion opening. The snapping member and the insertion opening cooperatively serve as the first connecting portion. The clamping member is provided with a claw at one end away from the rotating shaft. The claw serves as the second connecting portion. When the clamping member is moved to cause the claw to separate from the snapping member, the claw is aligned with the insertion opening, and the clamping member is able to be flipped.

Further, the claw is provided with a latching groove. The snapping member is provided with a protrusion corresponding to the latching groove. When the first connecting portion is engaged with the second connecting portion, the protrusion latches into the latching groove, or, the snapping member and the claw are each provided with a magnet member, when the first connecting portion is engaged with the second connecting portion, the snapping member and the claw are attracted by the magnet members.

Further, the connection assembly further includes an elastic member. When the clamping member is moved, the elastic member always resists one end of the clamping member having the rotating shaft.

An atomizer includes any of the above liquid injection protection devices.

Further, the atomizer includes a housing, a base assembly and an atomizing head. The connection assembly is arranged at one end of the housing. The base assembly is arranged at the other end of the housing opposite to the connection assembly. A liquid storage chamber in communication with the liquid injection hole is provided in the housing. The atomizing head is received in the liquid storage chamber.

An electronic cigarette includes any of the above atomizer.

The advantages of the present invention are described as follows:
In the liquid injection protection device provided by the present invention, when one end of the locking member extends through the locking hole to be connected to the connection assembly, the movement of the cover body is restricted by the locking member, so as to prevent the movement of the cover body to open the liquid injection hole. When a liquid injection needs to be performed, the locking member is required to be unlocked firstly, and then the cover body is moved to open the liquid injection hole. Because the liquid injection hole can only be opened by a complicated operation procedure, the children cannot open the liquid injection hole casually, and exposure or ingesting of the e-cigarrette liquid by the children can be avoided, so it is childproof. The present invention further provides an atomizer having the liquid injection protection device and an electronic cigarette having the atomizer.

### BRIEF DESCRIPTION OF THE DRAWINGS

The invention is specifically illustrated with reference to accompanying drawings and embodiments in the following description.
FIG. 1 is a schematic view of an atomizer of a first embodiment of the present invention (the cover body is in a flipped state);
FIG. 2 is a partially exploded view of the atomizer of FIG. 1;
FIG. 3 is an exploded view of the liquid injection protection device of the atomizer of FIG. 1 (the mouthpiece is not shown);
FIG. 4 is an exploded view of the liquid injection protection device of the atomizer of FIG. 3, viewed from another aspect;
FIG. 5 is a schematic view of the lining member of the liquid injection protection device of the atomizer of FIG. 3;
FIG. 6 is a cross-sectional view of the liquid injection protection device of the atomizer of FIG. 3 (the cover body is in a locked state);
FIG. 7 is another cross-sectional view of the liquid injection protection device of the atomizer of FIG. 3 (the cover body is in an unlocked state);
FIG. 8 is a cross-sectional view of the atomizer of FIG. 1 (the mouthpiece is not shown);
FIG. 9 is an enlarged view of the circled portion A of the atomizer of FIG. 2;
FIG. 10 is a schematic view of an atomizer of a second embodiment of the present invention (the cover body is in a flipped state);
FIG. 11 is a partially exploded view of the atomizer of FIG. 10;
FIG. 12 is an exploded view of the liquid injection protection device of the atomizer of FIG. 10 (the mouthpiece is not shown);
FIG. 13 is a cross-sectional view of the liquid injection protection device of the atomizer of FIG. 12 (the cover body is in a flipped state);
FIG. 14 is a cross-sectional view of the atomizer of FIG. 10;
FIG. 15 is a schematic view of an atomizer of a third embodiment of the present invention (the cover body is in a locked state);
FIG. 16 is a cross-sectional view of the atomizer of FIG. 15;
FIG. 17 is a schematic view of the atomizer of FIG. 15, showing another state (the cover body is in an unlocked state);
FIG. 18 is a schematic view of the atomizer of FIG. 15, showing a further state (the liquid injection hole is opened);
FIG. 19 is a schematic view of an atomizer of a fourth embodiment of the present invention (the cover body is in a locked state);
FIG. 20 is a cross-sectional view of the liquid injection protection device of the atomizer of FIG. 19;
FIG. 21 is a schematic view of the atomizer of FIG. 19, showing another state (the cover body is in an unlocked state);
FIG. 22 is a schematic view of the atomizer of FIG. 19, showing a further state (the liquid injection hole is opened);
FIG. 23 is a schematic view of an atomizer of a fifth embodiment of the present invention (the cover body is in a locked state);
FIG. 24 is a cross-sectional view of the liquid injection protection device of the atomizer of FIG. 23;
FIG. 25 is a schematic view of the atomizer of FIG. 23, showing another state (the cover body is in an unlocked state);
FIG. 26 is a schematic view of the atomizer of FIG. 23, showing a further state (the liquid injection hole is opened);
FIG. 27 is a schematic view of an atomizer of a sixth embodiment of the present invention (the cover body is in a locked state);

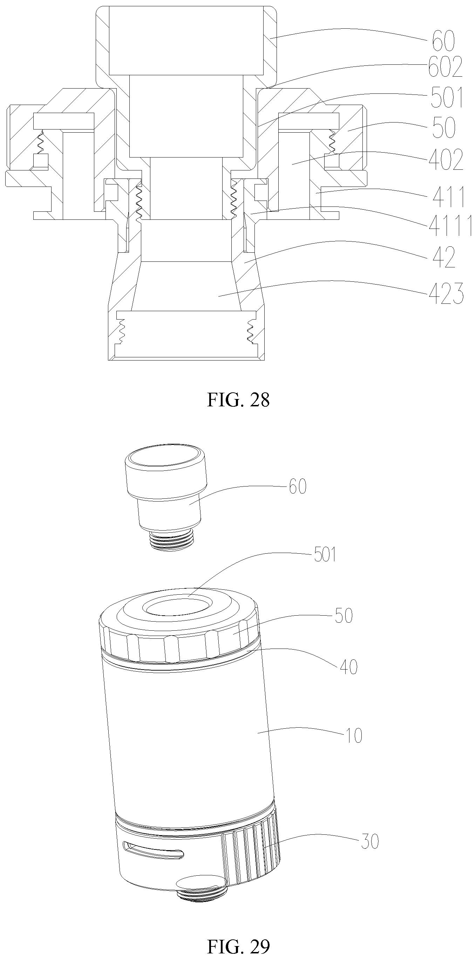

FIG. 28 is a cross-sectional view of the liquid injection protection device of the atomizer of FIG. 27;
FIG. 29 is a schematic view of the atomizer of FIG. 27, showing another state (the cover body is in an unlocked state);
FIG. 30 is a schematic view of the atomizer of FIG. 27, showing a further state (the liquid injection hole is opened).

The following table lists the various components and reference numerals of the figures.

| | | |
|---|---|---|
| atomizer 100,200,300,400,500,600 | | housing 10 |
| locking groove 4163 | liquid storage chamber 101 | atomizing head 20 |
| atomizing sleeve 21 | second electrode 22 | atomizing chamber 201 |
| liquid inlet hole 202 | base assembly 30 | bottom seat 31 |
| first electrode 32 | electrode sleeve 33 | air adjusting ring 34 |
| connecting tube 424, 311 | first insulating member 35 | second insulating member 36 |
| connection assembly 40 | smoke outlet hole 401 | liquid injection hole 402 |
| connection base 411 | lining member 414 | stopping member 415 |
| connecting post 4111 | perforation 4142, 4241 | embedding groove 4144 |
| first convex 4145 | second convex 4146 | slot 4147 |
| snapping member 420 | protrusion 4181 | clamping groove 4151 |
| insertion hole 4152 | hollow chamber 4153 | insertion opening 4154 |
| restricting groove 4155 | sliding groove 4156, 4165 | elastic member 419 |
| clamping portion 4191 | resisting portion 4192 | gland 416 |
| sealing ring 418 | cutout 4161 | connecting wall 4162 |
| sidewall 4164 | recess 4166 | covering portion 432 |
| movable member 433 | sealing groove 4143 | pin 417 |
| ventilation member 42 | restricting recess 601 | restricting protrusion 5011 |
| first fixing member 431 | second fixing member 435 | mouthpiece 60 |
| smoke outlet passage 423 | sliding member 46 | cover body 50 |
| through hole 501 | clamping member 434 | plug portion 4331 |
| aligning hole 4341 | sealing pad 45 | first connecting member 4342 |
| claw 4343 | latching groove 4344 | second connecting portion 421 |
| first connecting portion 422 | second connecting member 4345 | resisting edge 602 |
| shaft hole 4347 | rotating shaft 436, 503 | movable ring 403 |
| sealing member 502 | rotating member 44 | guiding groove 4149 |

### DETAILED DESCRIPTION OF EMBODIMENTS

In order to facilitate the understanding of the present invention, the present invention will be described more fully below with reference to the accompanying drawings. Preferred embodiments of the invention are shown in the drawings. However, the invention may be embodied in many different forms and is not limited to the embodiments described herein. Rather, these embodiments are provided so that the understanding of the disclosure of the invention may be more thorough.

It should be noted that when an element is referred to as being "fixed" to another element, it may be directly on the another element or intervening elements may be present. When an element is referred to be "connected" to another element, it may be connected to the another element directly or through intervening elements.

All technical terms used herein have the same meaning as commonly understood by one of ordinary skill in the art to the present invention. The terminology used in the present invention is for the purpose of describing particular embodiments and is not intended to limit the invention. The term "and/or" used herein includes any and all combinations of one or more of the associated listed items.

### First embodiment

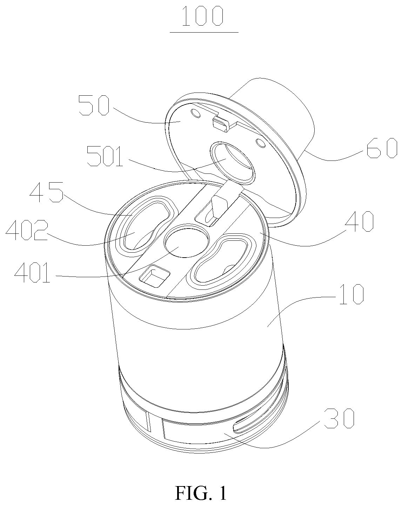

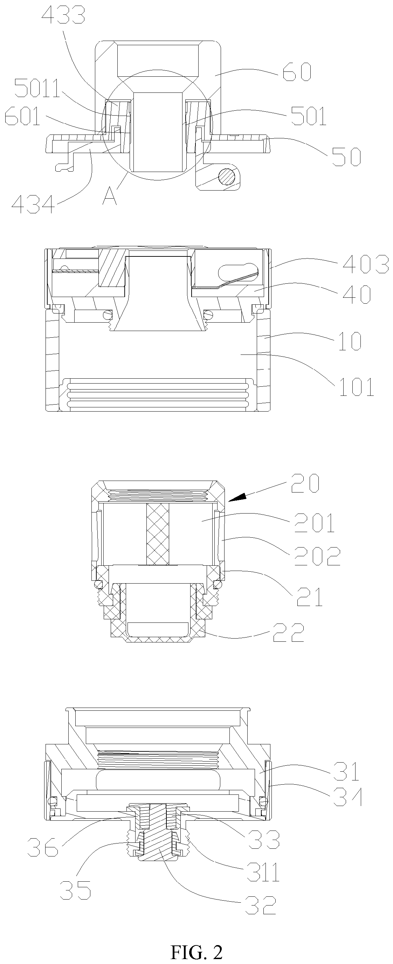

Referring to FIG. 1 and FIG. 2, an electronic cigarette is provided by the first embodiment of the present invention. The electronic cigarette includes an atomizer 100 and a battery device (not shown) electrically connected to the atomizer 100. The atomizer 100 includes a housing 10, an atomizing head 20 received in the housing 10, a base assembly 30 connected to one end of the housing 10, a connection assembly 40 connected to the other end of the housing 10 opposite to the base assembly 30, and a cover body 50 covering the connection assembly 40. The battery device is detachably mounted on the base assembly 30 and is electrically connected to the atomizing head 20. The housing 10 stores e-cigarette liquid therein, and can supply the e-cigarette liquid to the atomizing head 20. The atomizing head 20 is driven by the battery device to heat the e-cigarette liquid to generate smoke for user to inhale.

The housing 10 has a substantially hollow cylindrical structure having openings at opposite ends. The housing 10 is provided with a liquid storage chamber 101 configured to storage the e-cigarette liquid. In the illustrated embodiment, the interior chamber of the housing 10 is the liquid storage chamber 101. The housing 10 is made of transparent or translucent materials, allowing user to observe an amount of the e-cigarette liquid in the liquid storage chamber 101 through the housing 10, for facilitating the user to perform e-cigarette liquid injection timely. In the illustrated embodiment, the housing 10 is made of glass. It is understood that, in an alternative embodiment not shown, the housing 10 can be further sleeved with a protective sleeve made of rigid materials such as stainless steel, for protecting the housing 10.

The base assembly 30 includes a bottom seat 31 mounted on a lower end of the housing 10, a first electrode 32 and an electrode sleeve 33 mounted on the bottom seat 31, and an air adjusting ring 34 rotatably sleeved on an outer side of the bottom seat 31.

Referring to FIG. 2 again, the bottom seat 31 has a substantially hollow cylindrical structure with an opening at an upper end thereof. The bottom seat 31 is provided with a connecting tube 311 in communication with an interior chamber of the bottom seat 31. The connecting tube 311 extends downwardly from a center of a bottom portion of the bottom seat 31. The first electrode 32 is mounted within the connecting tube 311. The electrode sleeve 33 is sleeved on the upper end of the first electrode 32, and is electrically connected to the atomizing head 20. Specifically, the battery device is connected to the connecting tube 311 of the bottom seat 31 via threads, enabling the first electrode 32 to be electrically connected to the battery device, thereby enabling the battery device to provide power for the atomizing head 20. In the illustrated embodiment, a first insulating member 35 is mounted between the first electrode 32 and the connecting tube 311, and a second insulating member 36 is mounted between the electrode sleeve 33 and the connecting tube 311, thereby realizing an insulating function. The first insulating member 35 and the second insulating member 36 are made of adiabatic materials. It is understood that, the first insulating member 35 and the second insulating member 36 include, but not limited to silicone or rubber.

The air adjusting ring 34 has a substantially cylindrical structure with two ends being open. The circumferential wall of the bottom seat 31 is provided with an air intake hole (not shown) in fluid communication with the interior chamber of the bottom seat 31. The circumferential wall of the air adjusting ring 34 is provided with an air regulating hole (not shown) corresponding to the air intake hole, and the air regulating hole is in communication with the outside environment. When the air adjusting ring 34 is rotated, a communicating area between the air regulating hole and the air intake hole can be adjusted, for facilitating the user to adjust the air intake amount according to requirement.

The atomizing head 20 includes an atomizing sleeve 21 received in the liquid storage chamber 101, a heating assembly (not shown) received in the atomizing sleeve 21, and a second electrode 22 mounted on a bottom of the atomizing sleeve 21. The upper end and the lower end of the atomizing sleeve 21 are in communication with each other, and an atomizing chamber 201 is provided in the atomizing sleeve 21. The atomizing sleeve 21 is provided with a liquid inlet hole 202 communicating the liquid storage chamber 101 with the atomizing chamber 201. The heating assembly is positioned within the atomizing chamber 201. The heating assembly includes a heating member (not shown) and a liquid guiding member (not shown) which contact each other. The liquid guiding member is configured to absorb the e-cigarette liquid in the liquid storage chamber 101 via the liquid inlet hole 202. The heating member is configured to heat the e-cigarette liquid absorbed on the liquid guiding member, causing the e-cigarette liquid to generate smoke. The heating member can be a heating wire, the liquid guiding member can be a liquid guiding cotton. With regard to the setting mode between the heating member and the liquid guiding member, the liquid guiding cotton can wrap on the heating wire, or the heating wire can wrap on the liquid guiding cotton. Further, the lower end of the second electrode 22 contacts with and is electrically connected to the electrode sleeve 33, and the upper end of the second electrode 22 is electrically connected to the heating member, thereby enabling the battery device to provide power for the heating member.

Referring to FIG. 1 again, the connection assembly 40 is assembled to an upper end of the housing 10. The cover body 50 can move between a locked position and an unlocked position relative to the connection assembly 40. The connection assembly 40 includes a smoke outlet hole 401 in communication with the atomizing chamber 201 and a liquid injection hole 402 in communication with the liquid storage chamber 101. The cover body 50 is provided with a through hole 501. When the cover body 50 is in the locked position relative to the connection assembly 40, the cover body 50 covers the connection assembly 40 and cannot be flipped relative to the connection assembly 40, the through hole 501 is in communication with the smoke outlet hole 401, and the liquid injection hole 402 is sealed. When the cover body 50 is moved to the unlocked position from the locked position relative to the connection assembly 40 through a sliding pushing by an external force, the cover body 50 can be flipped relative to the connection assembly 40, the liquid injection hole 402 is opened.

Therefore, when the electronic cigarette is in use, the though hole 501 of the cover body 50 is in communication with the smoke outlet hole 401, facilitating the smoke atomized and generated in the atomizing chamber 201 to flow out via the through hole 501, for user to inhale. At this time, the liquid injection hole 402 is sealed, and a liquid injection operation cannot be performed. When a liquid injection operation is required, the cover body 50 is moved to the unlocked position from the locked position via a sliding pushing, and then flipped relative to the connection assembly 40, allowing the liquid injection hole 402 to be opened, so that the user can perform a liquid injection operation.

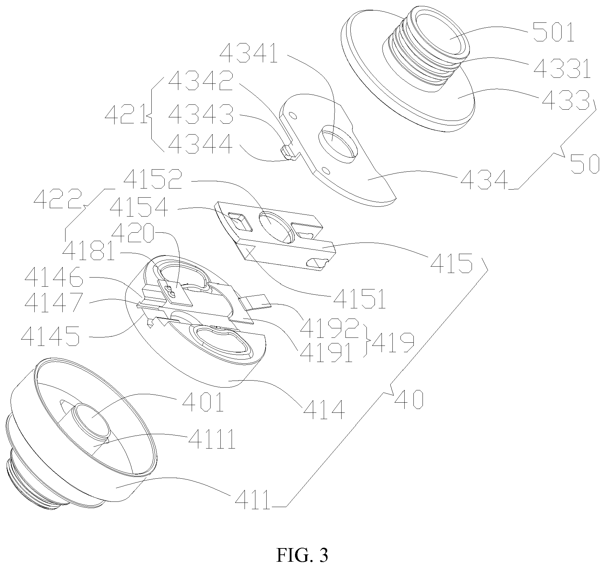

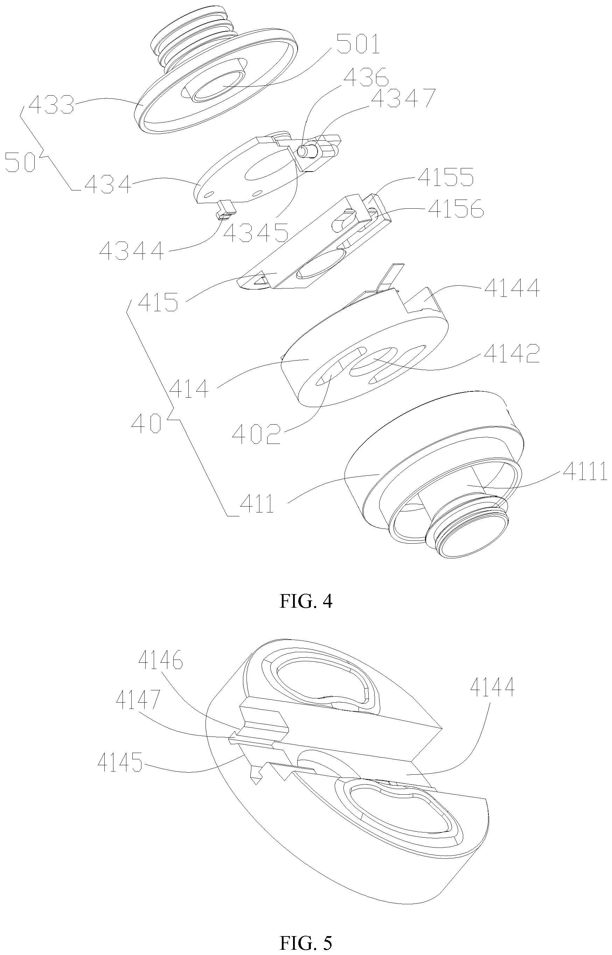

Specifically, referring to FIG. 3 and FIG. 4 at the same time, in the illustrated embodiment, the connection assembly 40 includes a connection base 411, a lining member 414 received in the connection base 411, and a stopping member 415 embedded in the lining member 414.

The connection base 411 has a substantially hollow cylindrical structure with an opening at an upper end. The connection base 411 is arranged on one end of the housing 10 away from the base assembly 30. The connection base 411 is provided with a connecting post 4111 on a bottom surface, the connecting post 4111 is hollow and passes through the bottom surface of the connection base 411. One end of the connecting post 4111 located below the bottom surface of the connection base 411 is connected to the atomizing sleeve 21 via threads, for fixing the connection assembly 40 to the atomizing head 20 and permitting a ventilation of the smoke in the atomizing chamber 201. One end of the connecting post 4111 away from the atomizing head 20 is received in the connection base 411. The smoke outlet hole 401 is formed by a port of the one end of the connecting post 4111 away from the atomizing head 20.

The lining member 414 has a substantially cylindrical structure. The lining member 414 is provided with a perforation 4142 at a center. The perforation 41042 extends through upper and lower surfaces of the lining member 414. The lining member 414 is received in the connection base 411. When the lining member 414 is received in the connection base 411, one end of the connecting post 4111 received in the connection base 411 extends through the perforation 4142. The lining member 414 is provided with an embedding groove 4144 at a surface of the lining member 414 facing the cover body 50. The embedding groove 4144 is arranged along a radial direction of the lining member 414. The embedding groove 4144 is configured to mount the stopping member 415, for fixing the stopping member 415. The embedding groove 4144 has a substantially elongated structure, and opposite ends of the embedding groove 4144 pass through the circumferential wall of the lining member 414. A width of the embedding groove 4144 is greater than a diameter of the perforation 4142, the embedding groove 4144 is in communication with the perforation 4142. Referring to FIG. 5, a bottom wall of one end of the embedding groove 4144 is provided with a first convex 4145, the embedding groove 4144 is provided with two second convexes 4146 on opposite sidewalls adjacent to the first convex 4145. Each of the two second convexes 4146 is provided with a slot 4147 at a bottom portion, each slot 4147 passes through two sidewalls of a corresponding second convex 4146, and the two sidewalls are arranged along a length direction of the embedding groove 4144. An insertion groove (not labeled) is constituted by a space formed between the top surface of the first convex 4145 and the upper wall of the slot 4147.

In the illustrated embodiment, the number of the liquid injection holes 402 is two, the two liquid injection holes 402 are arranged on opposite sides of the embedding groove 4144. The liquid injection hole 402 passes through upper and lower surfaces of the lining member 414. It is understood that, in an alternative embodiment not shown, the number of the liquid injection hole 402 is at least one, and the positions of the liquid injection hole 402 is not limited, so long as the liquid injection can be finished. In the illustrated embodiment, the connection base 411 is provided with a passing hole (not shown) in communication with the liquid injection hole 402. The liquid injection hole 402 is in communication with the liquid storage chamber 101 via the passing hole.

The connection assembly 40 further includes a snapping member 420 having a substantially sheet structure. The snapping member 420 is tightly engaged in the insertion groove. When the snapping member 420 is assembled, the snapping member 420 is merely inserted into the insertion groove along the opening end of the insertion grove. In the illustrated embodiment, the snapping member 420 is provided with two protrusions 4181 protruding from a surface of the snapping member 420 facing the cover body 50. The two protrusions 4181 are made of elastic materials. When the protrusion 4181 is exerted by an external force, the protrusion 4181 can be deformed, and when the external force is removed, the protrusion 4181 can reset.

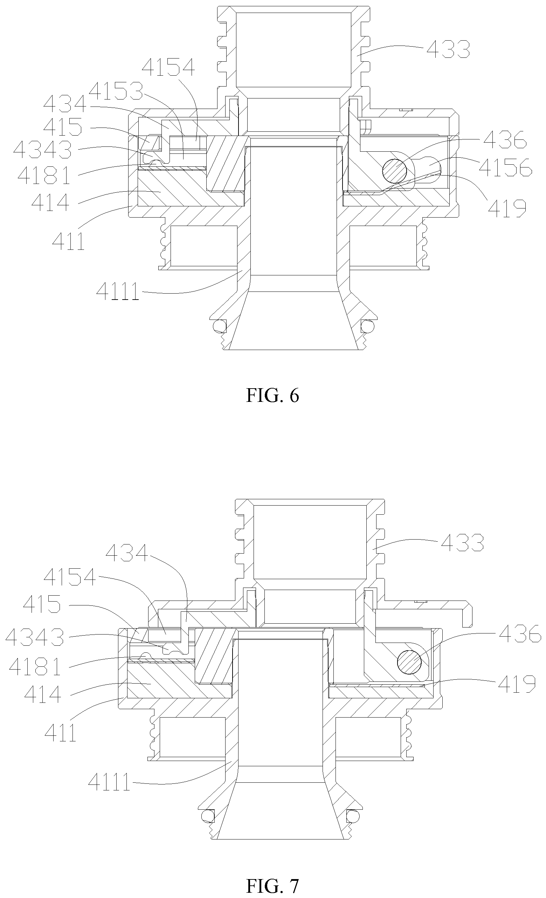

The stopping member 415 has a substantially elongated shape. The stopping member 4151 is provided with a clamping groove 415 corresponding to the second protrusion 4181, the clamping groove 4151 is recessed from the surface of the stopping member 415 away from the cover body 50. The stopping member 415 is provided with an insertion hole 4152 corresponding to the connecting post 4111. The insertion hole 4152 passes through upper and lower surfaces of the stopping member 415. Referring to FIG. 6, when the stopping member 415 is embedded in the embedding groove 4144, the connecting post 4111 is inserted into the insertion hole 4152, a bottom wall of the clamping groove 4151 abuts on the second convex 4146. Thus, a hollow chamber 4153 is formed between the stopping member 415 and the snapping member 420. It is obvious that, the hollow chamber 4153 is a portion of the clamping groove 4151. In the illustrated embodiment, the stopping member 415 is provided with an insertion opening 4154 on a surface of the stopping member 415 facing the cover body 50, the insertion opening 4154 is in communication with the hollow chamber 4153. It is understood that, the insertion opening 4154, the snapping member 420 and the protrusion 4181 cooperatively serve as a first connecting portion 422.

The stopping member 415 is provided with a restricting groove 4155 at one end opposite to the insertion opening 4154. The restricting groove 4155 is arranged along a length direction of the stopping member 415, and passes through the upper surface and the lower surface of the stopping member 415. Opposite sidewalls of the restricting groove 4155 are provided with sliding grooves 4156 along a length direction of the stopping member 415, and the sliding groove 4156 has an elongated shape.

In the illustrated embodiment, the connection assembly 40 further includes an elastic member 419 clamped between the lining member 414 and the stopping member 415. The elastic member 419 includes a clamping portion 4191 and a resisting portion 4192 connected to the clamping portion 4191. The clamping portion 4191 has a substantially square shape, the resisting portion 4192 has a substantially elongated structure. The clamping portion 4191 is clamped between the lining member 414 and the stopping member 415. The resisting portion 4192 is connected to one side of the clamping portion 4191 away from the insertion hole 4152, and the resisting portion 4192 is inclined along a direction away from the bottom wall of the embedding groove 4144. The resisting portion 4192 processes elasticity. When the resisting portion 4192 is pressed downwardly, one end of the resisting portion 4192 away from the clamping portion 4191 can move downwardly, and when the pressure is removed, the resisting portion 4192 can reset. In the illustrated embodiment, the clamping portion 4191 and the resisting portion 4192 can be an integrally formed stainless steel elastic sheet. It is understood that other elastic members such as spring can also be applied.

Referring to FIG. 3 and FIG. 4 again, the cover body 50 includes a movable member 433 and a clamping member 434 positioned under the movable member 433.

Specifically, the movable member 433 has a substantially disc structure. The through hole 501 is provided in the center of the movable member 433, and the sidewall of the through hole 501 forms a plug portion 4331 extending along a direction away from the connection assembly 40.

The clamping member 434 has a substantially semi-circular shape. The clamping member 434 is fixedly assembled to the surface of the movable member 433 away from the plug portion 4331. When the cover body 50 is in the locked position, the clamping member 434 can cover the liquid injection hole 402, thereby sealing the liquid injection hole 402. The clamping member 434 is provided with an aligning hole 4341 corresponding to the through hole 501. When the clamping member 434 is connected to the movable member 433, the aligning hole 4341 is in communication with the through hole 501. In the embodiment, the clamping member 434 is inserted in the movable member 433. It is understood that, in an alternative embodiment not shown, the clamping member 434 can be connected to the movable member 433 via latching or adhering, or the clamping member 434 and the movable member 433 may be integrally formed, which is not limited herein.

Referring to FIG. 1 again, in order to improve the sealing effect of the liquid injection hole 402 sealed by the clamping member 434, a sealing pad 45 is arranged on a periphery of the liquid injection hole 402.

The clamping member 434 is provided with a first connecting member 4342 protruding downwardly from the surface of the clamping member 434 opposite to the movable member 433. The first connecting member 4342 is located adjacent to the insertion opening 4154 of the stopping member 415. The first connecting member 4342 forms a claw 4343 extending outwards from one end away from the clamping member 434. The claw 4343 is provided with a latching groove 4344 recessed from a surface opposite to the clamping member 434. The latching groove 4344 is used for latching with the protrusion 4181. The claw 4343 can be inserted into the hollow chamber 4153 through the insertion opening 4154. When the clamping member 434 performs a sliding movement, the claw 4343 can be driven by the clamping member 434 to move in the hollow chamber 4153, thereby enabling the protrusion 4181 to latch into the latching groove 4344, or enabling the protrusion 4181 to be separated from the latching groove 4344. It is understood that, the first connecting member 4342, the claw 4343 and the latching groove 4344 cooperatively serve as a second connecting portion 421. When the cover body 50 is in the locked position, the first connecting portion 422 engages with the second connecting portion 421. At this time, the protrusion 4181 is latched into the latching groove 4344, the circumference of the insertion opening 4154 abuts against the claw 4343, thereby restricting a flipping action of the clamping member 434 relative to the connection assembly 40. In the illustrated embodiment, the protrusion 4181 is made of an elastic material, the protrusion 4181 is deformed and latched into the latching groove 4344. It is understood that, in an alternative embodiment not shown, the claw 4343 is made of an elastic material, the claw 4343 is deformed to enable the protrusion 4181 to be latched into the latching groove 4344.

It is understood that, in an alternative embodiment not shown, the first connecting portion 422 and the second connecting portion 421 are each provided with a magnet member such as magnet. When the cover body 50 is in the locked position, the first connecting portion 422 and the second connecting portion 421 are magnetically connected. Specifically, the second connecting portion 421 includes a claw 4343, the claw 4343 is itself a magnet member or is provided with a magnet member. The first connecting portion 422 includes the snapping member 420 and the protrusion 4181. The protrusion 4181 is provided with a magnet member such as magnet. When the claw 4343 moves in place, the magnet member of the claw 4343 and the magnet member of the protrusion 4181 are attracted, thereby locking the clamping member 434 and improving an air tightness between the cover body 50 and the connection assembly 40. When the cover body 50 is in the unlocked position, the first connecting portion 422 is detached from the second connecting portion 421, the circumference of the insertion opening 4154 no longer abuts against the claw 4343, thereby allowing the cover body 50 to be able to be flipped. It is understood that, in alternative embodiments, each of the first connecting portion 422 and the second connecting portion 421 can adopt other shape, structure or connecting mode. For example, each of the first connecting portion 422 and the second connecting portion 421 can include a claw, the claw of the second connecting portion 421 can have a shape the same as that in the illustrated embodiment, but the claw of the first connecting member 4222 has an inversed shape from the claw 4343 of the second connecting portion 421, such that the claws can be clamped with each other.

It is understood that, the first connecting portion 422 and the second connecting portion 421 cooperatively serve as a second locking mechanism. When the cover body 50 is moved to allow the first connecting portion 422 to engage with the second connecting portion 421, the cover body 50 is locked. When the cover body 50 is moved to allow the first connecting portion 422 to disengage from the second connecting portion 421, the cover body 50 is unlocked.

The clamping member 434 is provided with a second connecting member 4345 protruding from a surface opposite to the movable member 433, and the second connecting member 4345 is located adjacent to the restricting groove 4155 of the stopping member 451. The second connecting member 4345 has an "L" structure. The second connecting member 4345 engages in the restricting groove 4155 and can slide within the restricting groove 4155. The second connecting member 4345 is provided with a shaft hole 4347 on its sidewalls. In the illustrated embodiment, the cover body 50 further includes a rotating shaft 436. The rotating shaft 436 is mounted in the shaft hole 4347 and is slidably engaged in the sliding grooves 4156. When the rotating shaft 436 is moved to reach one end of the sliding groove 4156 away from the insertion opening 4154, the cover body 50 can be flipped, and the cover body 50 is allowed to rotate around the rotating shaft 436 relative to the connection assembly 40. It should be noted that, when the rotating shaft 436 is located at any position along the sliding groove 4156, the resisting portion 4191 of the elastic member 419 elastically resists the second connecting member 4345.

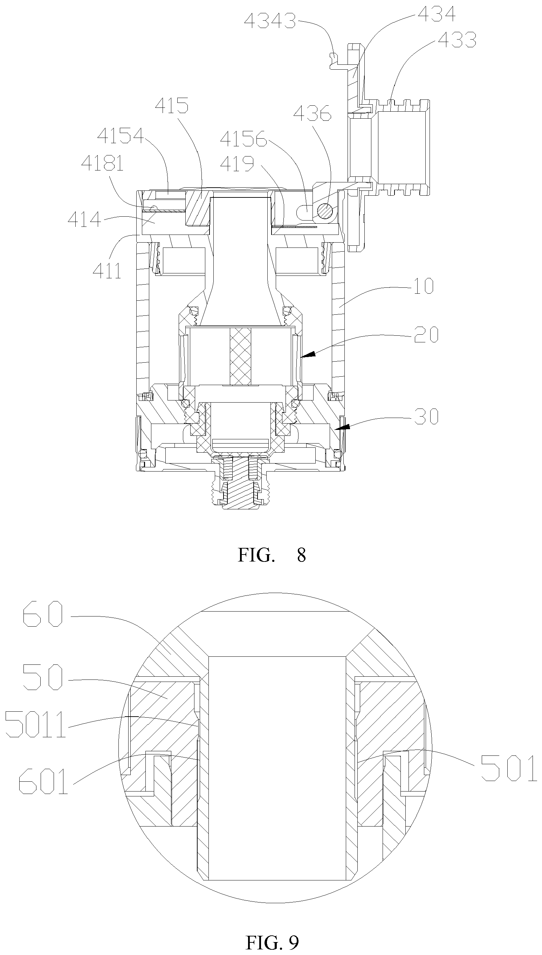

Referring to FIG. 7 and FIG. 8, when the user wants to perform a liquid injection, the cover body 50 is firstly moved away from the insertion opening 4154 through a sliding pushing, enabling the clamping member 434 to be driven by the movable member 433 to move, such that the first connecting portion 422 and the second connecting portion 421 are unlocked. For example, the protrusion 4181 is disengaged from the latching groove 4344 of the claw 4334. When the claw 4343 is aligned with the insertion opening 4154, at this time, the rotating shaft 436 reaches one end of the sliding groove 4156 away from the insertion opening 4154, and the cover body 50 is in the unlocked position. Then, the cover body 50 is flipped to open the liquid injection hole 402, so that the user can thereby perform a liquid injection operation. Referring to FIG. 6, when the liquid injection is finished and the liquid injection hole 402 is required to be closed, the cover body 50 is flipped back firstly, causing the claw 4343 to extend into and be received in the hollow chamber 4153 via the insertion opening 4154, at this time, the cover body 50 is still in the unlocked position. The cover body 50 is then moved towards the protrusion 4181 via a sliding pushing, enabling the latching groove 4344 to latch with the protrusion 4181, the circumference of the insertion opening 4154 abuts against the claw 4343, thereby preventing a flipping action of the clamping member 43 relative to the connection assembly 40. That is, the cover body 50 cannot be flipped relative to the connection assembly 40. At this time, the cover body 50 is in the locked position, the first connecting portion 422 and the second connecting portion 421 are interlocked, the cover body 50 covers the connection assembly 40, and the liquid injecting hole 402 is sealed. The user can perform smoking, and cannot perform a liquid injection operation.

It should be noted that, in the illustrated embodiment, when the cover body 50 is in the locked position, on one hand, the elastic member 419 has a resisting action to the second connecting member 4345, one the other hand, a latching action is existed between the latching groove 4344 and the protrusion 4181, thereby permitting the cover body 50 to stay in the locked position all the time. The situation that the liquid filling cover 43 slides to the unlocked position easily due to a small locking force is avoided. It is understood that in other embodiments not shown, the presence of the elastic member 419 and the engagement of the latching groove 4344 and the projection 4181 can be retained only one, to also achieve the effect of preventing the liquid filling cover 43 from sliding easily.

In the illustrated embodiment, the atomizer 100 further includes a first locking mechanism. It is understood that, the connection assembly 40, the cover body 50, the first locking mechanism and the second locking mechanism cooperatively serve as a liquid injection protection device. The first locking mechanism includes a locking hole provided in the cover body 50 and a locking member capable of extending through the locking hole to be detachably connected to the connection assembly 40. When one end of the locking member extends through the locking hole to be connected to the connection assembly 40, a movement of the cover body 50 is restricted by the locking member. Thus, the cover body 50 cannot move to open the liquid injection hole 402.

Referring to FIG. 2 and FIG. 9 again, in the illustrated embodiment, the locking hole is the through hole 501 provided in the movable member 433 of the cover body 50, the locking member is the mouthpiece 60 extending through the through hole 501 to be connected to the connection assembly 40. Specifically, the mouthpiece 60 has a substantially hollow cylindrical structure having two openings at opposite ends. One end of the mouthpiece 60 extends through the through hole 501 and is inserted into the insertion hole 4152 of the stopping member 415. In the illustrated embodiment, the sidewall of the through hole 501 is provided with a restricting protrusion 5011, the external circumferential wall of the mouthpiece 60 is provided with a restricting recess 601. The restricting protrusion 5011 is movably embedded in the restricting recess 601, thus avoiding the loss of the mouthpiece 60 caused by separation of the mouthpiece 60 and the cover body 50. Specifically, when the mouthpiece 60 is drawn outwardly to enable the restricting protrusion 5011 to resist one end of the restricting recess 601, one end of the mouthpiece 60 is separated from the insertion hole 4152 and is retracted into the through hole 501. At this time, a locking relationship between the connection assembly 40 and the cover body 50 is removed, the cover body 50 can be pushed to move relative to the connection assembly 40, and the cover body 50 can be then flipped to open the liquid injection hole 402, such that the user can perform a liquid injection operation. When the mouthpiece 60 is inserted inwardly to enable the restricting protrusion 5011 to resist the opposite end of the restricting recess 601, the mouthpiece 60 is inserted into the insertion hole 4152, thereby locking the connection assembly 40 and the cover body 50, at this time, the user can inhale via the mouthpiece 60, but cannot perform a liquid injection operation. It is understood that, in an alternative embodiment not shown, the restricting protrusion 5011 can be protruded from an external wall of the mouthpiece 60, the restricting recess 601 is recessed from the sidewall of the through hole 501 accordingly, the loss of the mouthpiece 60 can also be prevented.

Referring to FIG. 2 again, in the illustrated embodiment, the liquid injection protection device further includes a movable ring 403 rotatably sleeved on an outer side of the connection assembly 40, and the connection assembly 40 is hidden within the movable ring 403. Specifically, the movable ring 403 is rotatably sleeved on an outer side of the connection base 411, it is difficult for the user to remove the connection assembly 40 by handling the movable ring 403 to rotate the connection assembly 40 relative to the housing 10, thereby preventing the connection assembly 40 from being disassembled. Thus, the children cannot casually disassemble the connection assembly 40 to contact the e-cigarette liquid in the liquid storage chamber 101. In a specific embodiment, the movable ring 403 hides a circumferential surface of the connection base 411 completely, causing the user difficult to apply a force to the connection base 411. Only when the cover body 50 is flipped relative to the connection assembly 40, a force can be easily applied to the cover body 50 to remove the connection assembly 40. Thereafter, an operation such as replacing the atomizing head 20 can be performed.

It is understood that, when the user performs a liquid injection, the first locking mechanism between the cover body 50 and the connection assembly 40 needs to be firstly unlocked, causing the locking member to separate from the locking hole. Then, the second locking mechanism between the cover body 50 and the connection assembly 40 needs to be unlocked, causing the first connecting portion 422 to separate from the second connecting portion 421, so that the cover body 50 can be flipped to open the liquid inlet hole 402 of the connection assembly 40, and a liquid injection operation can thereafter be performed. After the liquid injection operation is finished, the locking procedure is the inverse of the unlocking procedure, which is not described herein again.

It is understood that, in an alternative embodiment not shown, only one of the first locking mechanism and the second locking mechanism can be configured.

In the atomizer 100 provided by the first embodiment of the present invention, the locking relationship between the connection assembly 40 and the cover body 50 should be removed firstly, and then the cover body 50 can open the liquid injection hole 402 by a combining operation of a sliding pushing and a flipping, thus the liquid injection operation can be performed. Therefore, the opening procedure of the liquid injection hole 402 is complicated, the liquid injection protection device has a childproof function.

In the electronic cigarette provided by the first embodiment of the present invention, all technical features of the aforementioned atomizer 100 are included, thus the electronic cigarette has technical advantages the same as those in the aforementioned atomizer 100.

### Second embodiment

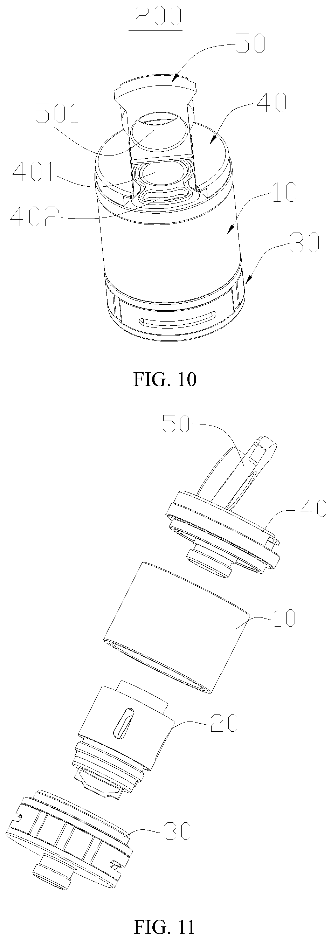

Referring to FIG. 10 and FIG. 11, an electronic cigarette is provided by the second embodiment of the present invention. The electronic cigarette includes an atomizer 200 and a battery device (not shown) electrically connected to the atomizer 200. Compared to the electronic cigarette of the first embodiment, the difference is that, the structure and the connecting mode of the connection assembly 40 and the cover body 50 in the atomizer 200 are different from the first embodiment, while the housing 10, the atomizing head 20 and the base assembly 30 are the same as the first embodiment, which are not specifically described herein.

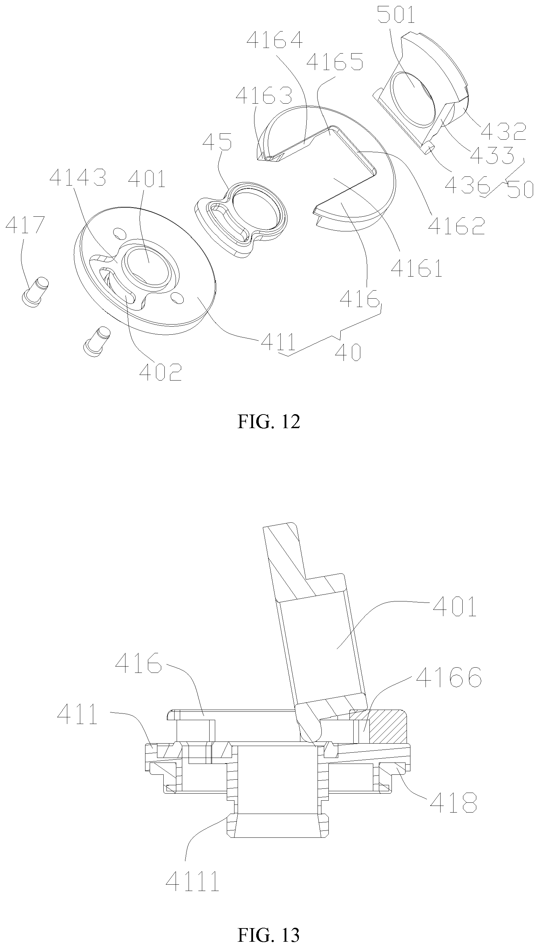

Referring to FIG. 12 and FIG. 13, in the illustrated embodiment, the connection assembly 40 includes a connection base 411 and a gland 416 covering the connection base 411. The smoke outlet hole 401 and the liquid injection hole 402 are sequentially provided in the connection base 411 from a center of the connection base 411 to an outside along a radial direction. The connection base 411 is arranged on one end of the housing 10 away from the base assembly 30. The connection base 411 is provided with a connecting post 4111. The connecting post 4111 is hollow and protrudes downwardly from a surface of the connection base 411 away from the gland 416. The connecting post 4111 is inserted into one end of the atomizing sleeve 21 away from the base assembly 30, for fixing the connection assembly 40 to the atomizing head 20, and allowing the smoke in the atomizing chamber 201 to flow out. Further, the connection assembly 40 further includes a sealing ring 418 positioned between the connection base 411 and the atomizing head 20, for preventing a leakage of the e-cigarette liquid.

The gland 416 is provided with a U-shaped cutout 4161 with an opening at an end, the U-shaped cutout 4161 is recessed from one side edge of the gland 416. The smoke outlet hole 401 and the liquid injection hole 402 are received in and exposed from the cutout 4161. Specifically, the cutout 4161 includes a connecting wall 4162 located at one side of the smoke outlet hole 401 away from the liquid injection hole 403 and two sidewalls 4164 connected to opposite ends of the connecting wall 4162 and extending in the same direction. The surface of the connecting wall 4162 adjacent to the connection base 411 is provided with a recess 4166, the recess 4166 is recessed from the connecting wall 4162 along a direction away from the smoke outlet hole 401, thereby a receiving gap having a determined height is formed between the gland 416 and the connection base 411. At the same time, each sidewall 4164 is provided with a sliding groove 4165 at one end of the sidewall 4164 connecting with the connecting wall 4162, the sliding groove 4165 is recessed from the surface of the sidewall 4164 adjacent to the connection base 411, and the sliding grooves provided at the two surfaces of the two sidewalls 4164 are disposed oppositely to each other. The sliding grooves 4165 are in communication with the cutout 4161 and the recess 4166. The two sliding grooves 4165 and the recess 4166 cooperatively form a U-shaped profile surrounding the cutout 4161 and having an opening direction the same as the cutout 4161.

When one end of the cover body 50 is received and restricted in the recess 4166, the cover body 50 is in the locked position, and the cover body 50 cannot be flipped relative to the connection assembly 40. When the cover body 50 is moved along the sliding grooves 4165 to enable the end of the cover body 50 to extend out of the recess 4166, the cover body 50 is in the unlocked position, and the cover body 50 can be flipped.

Specifically, the cover body 50 includes a movable member 433 and a rotating shaft 436 extending from opposite sides of one end of the movable member 433. The movable member 433 has a substantially elongated plate shape, it has a shape matching with the shape of the cutout 4161. The through hole 501 extends through the movable member 433, and the sidewall of the through hole 501 protrudes out of the surface of the movable member 433 away from the connection base 411. When the cover body 50 is in the locked position, the rotating shaft 436 is latched within the recess 4166, and the sidewall of the through hole 501 abuts against the sidewall of the recess 4166, thereby preventing the cover body 50 from flipping around the rotating shaft 436. When the cover body 50 is exerted by an external force, and moves toward the unlocked position from the locked position, the rotating shaft 436 moves into the sliding groove 4165 from the recess 4166, causing the sidewall of the through hole 501 and the sidewall of the cutout 4166 are separated from each other to form a rotation space for the movable member 433 to be flipped around the rotating shaft 436 relative to the gland 416. Thus, by a flipping of the cover body 50, the cover body 50 is opened relative to the connection assembly 40, to expose the liquid injection hole 402, for facilitating the user to conduct a liquid injection action. After the liquid injection is finished, the cover body 50 can be flipped to cover the connection assembly 40 and pushed back to the locked position from the unlocked position. When the movable member 433 is in the locked position, it cannot directly be flipped. When a sliding pushing is preformed to remove an interference between the movable member 433 and the cover 416, then the movable member 433 can be flipped, to prevent the cover body 50 from being opened during the use of the electronic cigarette. It is thus childproof.

The cover body 50 further includes a covering portion 432 arranged on one end of the movable member 433 opposite to the rotating shaft 436. The covering portion 432 extends to opposite sides of the movable member 433. A width of the covering portion 432 is greater than that of the cutout 4161. In the illustrated embodiment, the covering portion 432 has a sector shape extending to opposite sides of the movable member 433. At the same time, the gland 416 is provided with a locking groove 4163 at a bottom of one end of the gland 416 away from the connecting wall 4162, the locking groove 4163 is in communication with the cutout 4161. The locking groove 4163 has a width greater than that of the cutout 4161, i.e., the locking groove 4163 and the cutout 4161 cooperatively form a stepped groove with a wider top and a narrow bottom. Therefore, when the cover body 50 is pushed to the locked position relative to the connection assembly 40, the covering portion 432 is received in the locking groove 4163 and resists the bottom wall of the locking groove 4163, for preventing the cover body 50 to be pushed continuously. On the other hand, the upper surface of the covering portion 432 resists the upper wall of the locking groove 4163, to thereby further prevent flipping of the cover body 50 relative to the connection assembly 40. When the cover body 50 is pushed to the unlocked position relative to the connection assembly 40, the covering portion 432 is moved outside the locking groove 4163 through the opening of the locking groove 4163, enabling the cover body 50 to be capable of being flipped relative to the connection assembly 40.

It is understood that, the locking groove 4163 serves as a first connecting portion, and the covering portion 432 serves as a second connecting portion, the first connecting portion and the second connecting portion cooperatively serve as a second locking mechanism. When the cover body 50 is moved to cause the covering portion 432 to be received in the locking groove 4163, the cover body 50 is locked. When the cover body 50 is moved to cause the covering portion 432 to separate from the locking groove 4163, the cover body 50 is unlocked.

According to a specific embodiment, the liquid injection hole 402 is an arc-shaped opening surrounding partially the outer circumference of the smoke outlet hole 401. The size of the liquid injection hole 402 is increased to facilitate the liquid injection operation while being matched with the shape of the sector-shaped covering portion 432.

In the illustrated embodiment, in order to guarantee a tightness between the gland 416 and the cover body 50, a sealing groove 4143 is provided in the connection base 411 around a periphery of the smoke outlet hole 401 and the liquid injection hole 402. The connection assembly 40 further includes a sealing pad 45, the sealing pad 45 is received in the sealing groove 4143 and heretically seals the periphery of the smoke outlet hole 401 and the liquid injection hole 402.

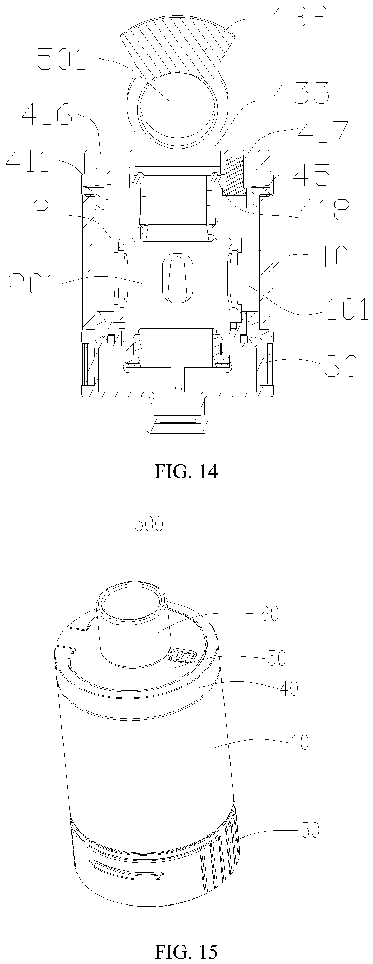

Referring further to FIG. 14, in the illustrated embodiment, the connection assembly 40 further includes a pin 417. The pin 417 extends through the connection base 411 from below and is fixed to the gland 416, for achieving a fixing relationship between the connection base 411 and the gland 416. It is understood that, in an alternative embodiment not shown, the fixing manner between the gland 416 and the connection base 411 can be varied according to requirement, or the two elements are integrally formed, which is not limited herein.

Further, to prevent opening the liquid injection hole 402 casually to contact the e-cigarette liquid in the liquid storage chamber 101 when the children have got the atomizer 200, the atomizer 200 can further include a first locking mechanism in addition to the second locking mechanism. The structure and arrangement of the first locking mechanism is the same as the first embodiment, which is not specifically described herein.

It is understood that, the connection assembly 40, the cover body 50, the first locking mechanism and the second locking mechanism cooperatively serve as a liquid injection protection device. When one end of the locking member extends through the locking hole to be connected to the connection assembly 40, a movement of the cover body 50 is restricted by the locking member, thereby preventing the movement of the cover body 50 to open the liquid injection hole 402.

It is understood that, when the user performs a liquid injection, the first locking mechanism between the cover body 50 and the connection assembly 40 is required to be removed firstly, enabling the locking member to separate from the locking hole, and then the second locking mechanism between the cover body 50 and the connection assembly 40 is removed, enabling the covering portion 432 to separate from the locking groove 4163, so that the liquid injection can be performed.

In the atomizer 200 provided by the second embodiment of the present invention, the locking relationship between the connection assembly 40 and the cover body 50 by the locking member is required to be removed firstly, and then the cover body 50 can open the liquid injection hole 402 by a combining operation of a sliding pushing and a flipping. Therefore, the opening procedure of the liquid injection hole 402 is relatively complicated, it therefore is childproof. At the same time, compared to the first embodiment, the atomizer 200 of the second embodiment 200 has a relatively simple structure, which facilitates to manufacture.

In the electronic cigarette provided by the second embodiment of the present invention, all the technical features in aforementioned atomizer 200 are included, thus the electronic cigarette has technical advantages the same as the atomizer 200.

### Third embodiment

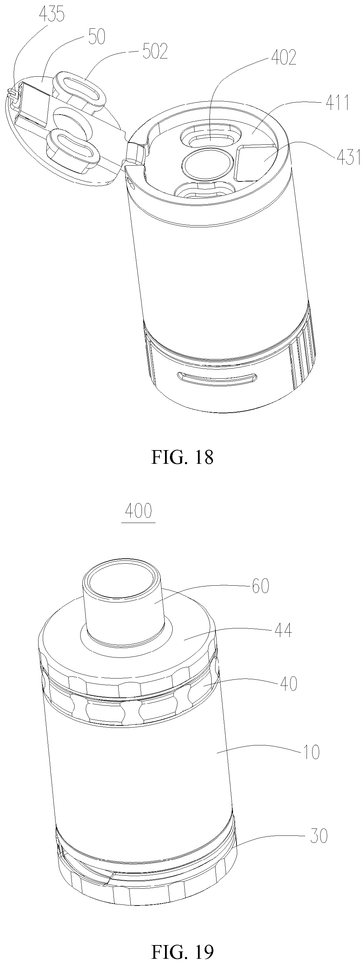

Referring to FIG. 15 and FIG. 18, an electronic cigarette is provided by the third embodiment of the present invention. The electronic cigarette includes an atomizer 300 and a battery device (not shown) electrically connected to the atomizer 300. The atomizer 300 includes a housing 10, an atomizing head 20 received in the housing 10, a base assembly 30 connected to one end of the housing 10, a connection assembly 40 connected to the other end of the housing 10 opposite to the base assembly 30, and a cover body 50 covering the connection assembly 40. The battery device is detachably mounted on the base assembly 30 and is electrically connected to the atomizing head 20. The housing 10 stores the e-cigarette liquid therein, and can supply the e-cigarette liquid to the atomizing head 20. The atomizing head 20 is driven by the battery device to heat the e-cigarette liquid to generate smoke for the user to inhale.

The structures of the housing 10, the atomizing head 20 and the base assembly 30 are the same as the first embodiment, which is not specifically described herein.

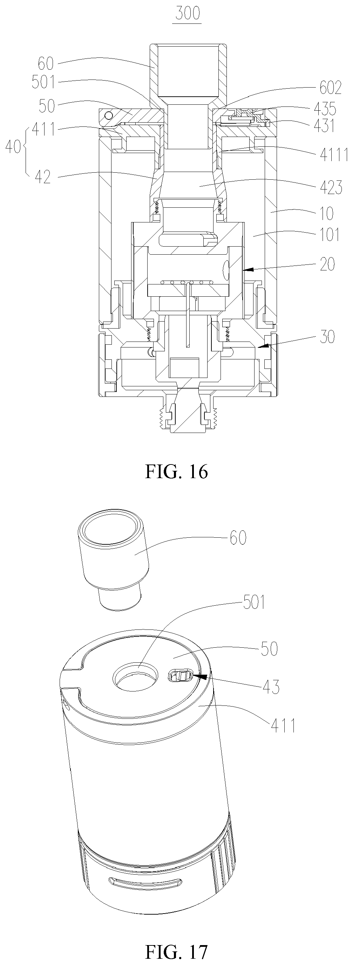

Referring to FIG. 16, in the illustrated embodiment, the connection assembly 40 includes a connection base 411 connected to an upper end of the housing 10, and a ventilation member 42 positioned within the housing 10 and connected to the lower end of the connection base 411.

The connection base 411 is inserted in the upper end of the housing 10. The connection base 411 has a substantially hollow cylindrical structure with an opening at an upper end. The connection base 411 is provided with a connecting post 4111 at the center of a bottom of the connection base 411 for the smoke to flow out. The upper end of the connecting post 4111 extends through the bottom of the connection base 411, the lower end of the connecting post 4111 extends downwardly into the housing 10 along an axial direction of the atomizer 300. The bottom of the connection base 411 is provided with two liquid injection holes 402 on opposite sides of the connecting post 4111, both the two liquid injection holes 402 are in communication with the liquid storage chamber 101. The user can inject e-cigarette liquid into the liquid storage chamber 101 via the liquid injection holes 402. It is understood that, in an alternative embodiment not shown, the number of the liquid injection hole 402 can be one or more than two.

The ventilation member 42 has a substantially hollow cylindrical structure with two openings at opposite ends. The upper end of the ventilation member 42 is detachably connected to the connection base 411. The lower end of the ventilation member 42 is detachably connected to the atomizing head 20. Specifically, the upper end of the ventilation member 42 is tightly sleeved into the interior of the connecting post 4111, the lower end of the ventilation member 42 is connected to the atomizing head 20 via threads. The interior chamber of the ventilation member 42 forms a smoke outlet passage 423. When in use, the smoke generated in the atomizing head 20 flows into the user's mouth via the smoke outlet passage 423.

It is understood that, in an alternative embodiment not shown, the upper end of the ventilation member 42 can be connected to the connecting post 4111 via a threaded connection or latching, the lower end of the ventilation member 42 can be connected to the atomizing head 20 via a threaded connection or latching, which is not limited herein.

It is understood that, in an alternative embodiment not shown, the upper end of the ventilation member 42 is tightly sleeved on an outer side of the connecting post 4111, thus a connection between the ventilation member 42 and the connecting post 4111 can also be achieved.

It is understood that, in an alternative embodiment not shown, the ventilation member 42 can be omitted, at this time, the atomizing head 20 is directly and detachably connected to the connecting post 4111.

Referring to FIG. 16 again, the cover body 50 has a substantially disc structure, the edge of the cover body 50 is hinged to the top of the connection base 411, enabling the cover body 50 to be flipped relative to the connection base 411, thereby causing the cover body 50 to cover or open the connection base 411, and accordingly to cover or open the liquid injection hole 402.

In order to prevent opening the liquid injection hole 402 casually to contact the e-cigarette liquid in the liquid storage chamber 101 when the children have got the atomizer 300, the atomizer 300 can further include a first locking mechanism. The first locking mechanism includes a locking hole provided in the cover body 50 and a locking member capable of passing through the locking hole to be detachably connected to the connection assembly 40. When one end of the locking member extends through the locking hole to be connected to the connection assembly 40, the movement of the cover body 50 is restricted by the locking member, thereby preventing the movement of the cover body 50 to open the liquid injection hole 402.

In the illustrated embodiment, the locking hole is the through hole 501 provided in the center the cover body 50, and the locking member is the mouthpiece 60 extending through the through hole 501 to be connected to the connection assembly 40. Specifically, the mouthpiece 60 has a substantially hollow cylindrical structure with two openings at opposite ends. After the lower end of the mouthpiece 60 extends through the through hole 501 of the cover body 50, the lower end of the mouthpiece 60 is tightly sleeved within the interior of the ventilation member 42, thereby establishing a connection between the mouthpiece 60 and the connection assembly 40. It is understood that, in an alternative embodiment not shown, when the upper end of the ventilation member 42 is tightly sleeved on an outer side of the connecting post 4111, or when the ventilation member 42 is omitted, the lower end of the mouthpiece 60 is tightly sleeved within the interior of the connecting post 4111, thereby establishing a connection between the mouthpiece 60 and the connection assembly 40. It can also be understood that, in an alternative embodiment not shown, the mouthpiece 60 can be connected to the connection assembly 40 by a threaded connection or latching.

In the illustrated embodiment, the mouthpiece 60 has an outer diameter equal to an inner diameter of the though hole 501 of the cover body 50. Specifically, the outer diameter of the portion of the mouthpiece 60 inserted in the through hole 501 is equal to the inner diameter of the through hole 501. When the mouthpiece 60 is connected to the connection assembly 40, because the outer diameter of the portion of the mouthpiece 60 inserted in the through hole 501 is equal to the inner diameter of the through hole 501. Since there is no movable space between the mouthpiece 60 and the through hole 501, the cover body 50 cannot be flipped relative to the mouthpiece 60. Further, due to a connection relationship between the mouthpiece 60 and the connection assembly 40, the cover body 50 and the mouthpiece 60 together cannot be flipped relative to the connection base 411, and the cover body 50 is locked.

Further, the external wall of the mouthpiece 60 is provided with a resisting edge 602. When the mouthpiece 60 is connected to the connection assembly 40, the resisting edge 602 abuts against the cover body 50, so that the cover body 50 is restricted between the connection base 411 and the resisting edge 602, preventing the cover body 50 from being flipped, and the cover body 50 is locked. Further, the resisting edge 602 further provides a restricting function for the mounting of the mouthpiece 60. When the resisting edge 602 abuts against the cover body 50, it means that the mouthpiece 60 is mounted in place. In the illustrated embodiment, the resisting edge 602 is formed by a shrinkage of a neck of the mouthpiece 60. It is understood that, in an alternative embodiment not shown, the resisting edge 602 can also be formed by extending outwardly from the circumferential surface of the mouthpiece 60 along a radial direction of the mouthpiece 60.

It is understood that, in the two selections, the outer diameter of the portion of the mouthpiece 60 inserted in the through hole 501 is equal to the inner diameter of the through hole 501, or the mouthpiece 60 is provided with a resisting edge 602 for abutting against the cover body 50, at least one selection is required to be met. When the mouthpiece 60 extends through the through hole 501 to be connected to the connection assembly 40, the movement of the cover body 50 is restricted by the mouthpiece 60, and the cover body 50 is prevented to move to open the liquid injection hole 402. Only when the mouthpiece 60 is dismantled, the cover body 50 can be further moved to open the liquid injection hole 402. It is understood that, the outer diameter of the portion of the mouthpiece 60 inserted in the through hole 501 is equal to the inner diameter of the through hole 501, and the mouthpiece 60 is provided with a resisting edge 602 for abutting against the cover body 50, the two manners can also be applied to the electronic cigarette of the first embodiment and the second embodiment, such that only when the mouthpiece 60 is dismantled, the cover body 50 can be further moved to open the liquid injection hole 402.

It should be noted that, the mouthpiece is a conventional element for an atomizer, the user sucks the mouthpiece with mouth to smoke. In the illustrated embodiment, by an improvement of the mouthpiece 60, the mouthpiece 60 not only has a smoking function, but also locks the cover body 50, preventing the liquid injection hole 402 to be opened casually, such that merely a minor change to the structure without increase of other parts, a childproof function is realized, it is thus cost saving.

Referring to FIG. 16 and FIG. 18, in the illustrated embodiment, in order to improve a reliability of the childproof function, for preventing the cover body 50 to be flipped after the locking member is dismantled by the children, a second locking mechanism is provided between the connection base 411 and the cover body 50. Specifically, the second locking mechanism includes a first connecting portion and a second connecting portion, the first connecting portion is a first fixing member 431 provided on the connection base 411, and the second connecting portion is a second fixing member 435 provided on the cover body 50. In the illustrated embodiment, the first fixing member 431 is a latching groove provided on the bottom of the inner circumferential wall of the connection base 411, and the second fixing member 435 is a latching member slidably arranged at the edge of the cover body 50 corresponding to the latching groove. The latching member is partially exposed from the upper surface of the cover body 50, for the user to conduct a sliding action to the latching member, causing one end of the latching member to latch with or separate from the latching groove. When the cover body 50 is flipped and covers the connection base 411, by a latching between the latching member and the latching groove, the covering applied to the connection base 411 by the cover body 50 is maintained, and the cover body 50 is prevented from being flipped casually. Therefore, even if the locking member is dismantled, the cover body 50 cannot be flipped casually, and the second locking mechanism should be further unlocked if a liquid injection is required.

It is understood that, in an alternative embodiment not shown, the first fixing member 431 can also be a first magnet member arranged on the bottom of the connection base 411, the second fixing member 435 is a second magnet member arranged on the lower surface of the cover body 50 corresponding to the first magnet member. When the cover body 50 covers the connection base 411, the first magnet member and the second member are mutually attracted, thereby the covering applied to the connection base 411 by the cover body 50 is maintained.

Referring to FIG. 18, in order to improve a tightness of the liquid injection hole 402 sealed by the cover body 50, when the cover body 50 covers the connection base 411, the cover body 50 is provided with a sealing member 502 protruding from the lower surface of the cover body 50 corresponding to the liquid injection hole 402. When the cover body 50 covers the connection base 411, the sealing member 502 is capable of latching into the liquid injection hole 402 for sealing the liquid injection hole 402, thereby an e-cigarette liquid leakage is avoided. It is understood that, the sealing member 502 is made of a sealing material such as silicone or rubber, which is not limited herein. It is understood that, when the sealing member 502 latches into the liquid injection hole 402, by a latching between the sealing member 502 and the liquid injection hole 402, the covering of the cover body 50 applied to the connection base 411 is maintained. At this time, as the second locking mechanism, the first fixing member 431 and the second fixing member 435 can be omitted.

It is understood that, the cover body 50, the connection assembly 40, the first locking mechanism and the second locking mechanism cooperatively serve as a liquid injection protection device. When one end of the locking member extends through the locking hole to be connected to the connection assembly 40, the movement of the cover body 50 is restricted by the locking member, so as to prevent the movement of the cover body 50 to open the liquid injection hole 402.

In the atomizer 300 provided by the third embodiment of the present invention, when the user wants to perform a liquid injection, referring to FIG. 17, the mouthpiece 60 is required to be dismantled firstly, and then the latching relationship between the first fixing member 431 and the second fixing member 435 is removed. At this time, the locking relationship between the cover body 50 and the connection base 411 is removed, referring to FIG. 18, the cover body 50 is flipped, to enable the liquid injection hole 402 to be opened. Because the liquid injection hole 402 can only be opened by a complicated operation procedure, thus the children cannot open the liquid injection hole 402 casually, and a contact and ingestion of the e-cigarette liquid by the children can be avoided, it is thus childproof. It is understood that, as the configuration in the first embodiment, a restricting recess 601 is provided in the mouthpiece 60 or in the cover body 50, the other one is provided with a restricting protrusion 5011, by an engagement between the restricting recess 601 and the restricting protrusion 5011, a range of movement of the mouthpiece 601 is restricted, the mouthpiece 60 is prevented from loss. When a liquid injection is required, the mouthpiece 60 is merely drawn upwards to enable the lower end of the mouthpiece 60 to be retracted into the through hole 501.

In the electronic cigarette provided by the third embodiment of the present invention, all technical features of the aforementioned atomizer 300 are included, thus the electronic cigarette has technical advantages the same as those in the aforementioned atomizer 300.

### Fourth embodiment

Referring to FIG. 19 through FIG. 22, an electronic cigarette is provided by the fourth embodiment of the present invention. The electronic cigarette includes an atomizer 400 and a battery device (not shown) electrically connected to the atomizer 400. Compared to the electronic cigarette of the third embodiment, the difference is that, the structure and the connecting mode of the connection assembly 40 and the cover body 50 in the atomizer 400 are different from the third embodiment, while the housing 10, the atomizing head 20 and the base assembly 30 are the same as the third embodiment, which are not specifically described herein.

Specifically, in the illustrated embodiment, the connection assembly 40 includes a connection base 411 connected to the upper end of the housing 10 and a lining member 414 received in the connection base 411.

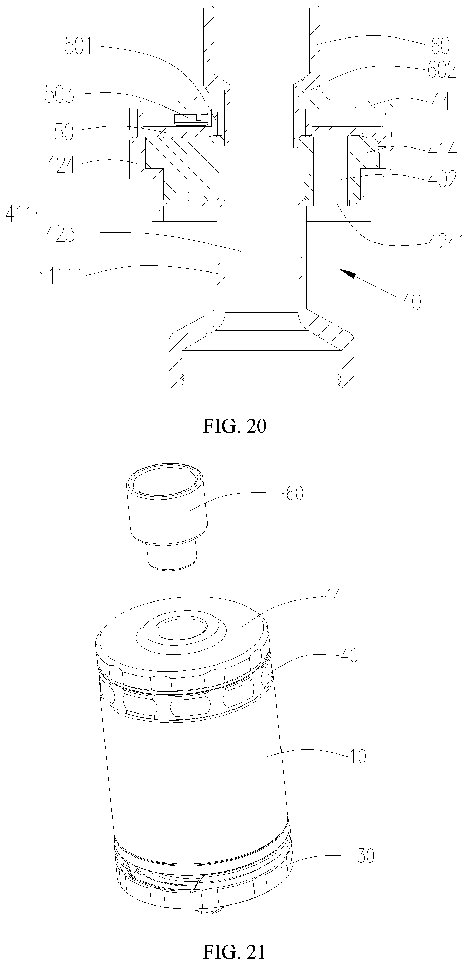

Referring to FIG. 20, in the illustrated embodiment, the connection base 411 includes a connecting tube 424 connected to an upper end of the housing 10 and a connecting post 4111 extending through the bottom of the connecting tube 424. The connecting tube 424 is provided with a receiving chamber with an opening at an upper end. The connecting tube 424 is provided with a perforation 4241 at a bottom, the perforation 4241 is arranged on one side of the connecting post 4111. The lower end of the connecting post 4111 extends into the housing 10 and is connected to the atomizing head 20, the interior chamber of the connecting post 4111 forms a smoke outlet passage 423, the smoke generated in the atomizing head 20 flows into the user's mouth via the smoke outlet passage 423.

The lining member 414 has a substantially cylindrical structure with opposite ends being opened. The lining member 414 is received in the connecting tube 424, the inner chamber of the lining member 414 is in communication with the smoke outlet passage 423. The lining member 414 is provided with a liquid injection hole 402 corresponding to the perforation 4241. The liquid injection hole 402 is in communication with the liquid storage chamber 101 through the perforation 4241. The user can inject the e-cigarette liquid into the liquid storage chamber 101 via the liquid injection hole 402.

Referring to FIG. 20, in the illustrated embodiment, the cover body 50 includes a cover plate having a substantially disc structure. A rotating shaft 503 is provided on the cover plate, the cover plate is rotatably mounted on the lining member 414 via the rotating shaft 503. A rotation center of the cover plate is staggered from a central axis of the lining member 414. During rotation, the lower surface of the cover plate contacts the upper surface of the lining member 414, and the cover plate can open or close the liquid injection hole 402.

In the illustrated embodiment, the cover body 50 further includes a rotating member 44 tightly sleeved on an outer side of the cover plate. When the rotating member 44 is rotated, the rotating member 44 drives the cover plate to rotate together. The rotating member 44 provides a shielding function for the cover plate, enabling the atomizer 400 to have an attractive appearance. It is understood that, in an alternative embodiment, the rotating member 44 can further be omitted, the user merely rotates the cover plate.

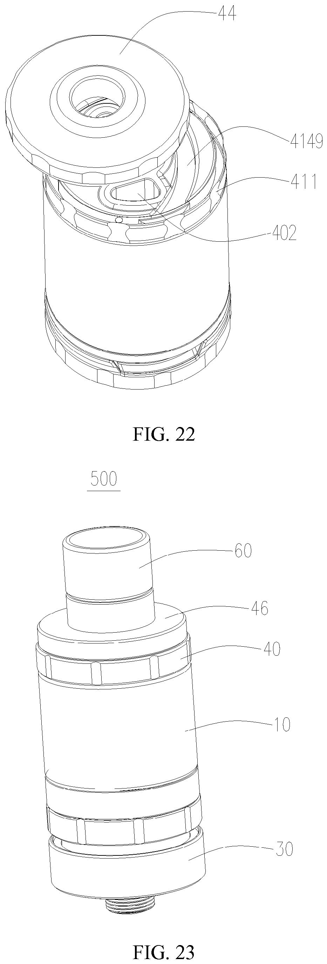

Referring to FIG. 22, when conducting a liquid injection, the rotating member 44 is rotated, the rotating member 44 drives the cover plate to rotate around the rotating shaft 503, and the cover plate is gradually staggered from the lining member 414, until the liquid injection hole 402 is exposed. At this time, the liquid injection hole 402 is opened, the user can perform a liquid injection operation.

In order to provide a stable rotation when the cover body 50 rotates, the lining member 414 is provided with an arc-shaped guiding groove 4149 around a center of the rotating shaft 503. The lower surface of the cover plate is provided with a guiding protrusion (not shown) corresponding to the guiding groove 4149. During the rotation of the cover body 50, the guiding protrusion can slide along the guiding groove 4149.

In order to improve the tightness of the liquid injection hole 402 sealed by the cover body 50, the cover plate is made of an elastic material such as silicone or rubber.

Further, the atomizer 400 further includes a first locking mechanism. It is understood that, the connection assembly 40, the cover body 50 and the first locking mechanism cooperatively serve as a liquid injection protection device. The first locking mechanism includes a locking hole provided in the cover body 50 and a locking member extending through the locking hole to be detachably connected to the connection assembly 40. In the illustrated embodiment, the locking hole is the through hole 501 provided in the cover body 50, the locking member is the mouthpiece 60 extending through the through hole 501 to be connected to the connection assembly 40. It is understood that, in the illustrated embodiment, since the cover plate is surrounded by the rotating member 44, therefore, the through hole 501 is provided in the rotating member 44.

The mouthpiece 60 has a substantially hollow cylindrical structure with two openings at opposite ends. The mouthpiece 60 extends through the through hole 501 of the cover body 50 to be tightly inserted in the lining member 414. At the same time, the liquid injection hole 402 is sealed by the cover body 50, and the mouthpiece 60 is in communication with the smoke outlet passage 423, the user can inhale by sucking the mouthpiece 60 with mouth.

When the mouthpiece 60 is connected to the connection assembly 40, a rotation of the cover body 50 is restricted by the mouthpiece 60. The mouthpiece 60 has an outer diameter equal to an inner diameter of the though hole 501 of the cover body 50. Specifically, the outer diameter of the portion of the mouthpiece 60 inserted in the through hole 501 is equal to the inner diameter of the through hole 501. Since there is no movable space between the mouthpiece 60 and the through hole 501, the cover body 50 cannot be flipped relative to the mouthpiece 60, and the cover body 50 is locked. When the mouthpiece 60 is dismantled, the cover body 50 is then rotatable.

Further, the external wall of the mouthpiece 60 is provided with a resisting edge 602. When the mouthpiece 60 is connected to the connection assembly 40, the resisting edge 602 abuts against the cover body 50, thereby providing a restricting function for the mounting of the mouthpiece 60. That is, when the resisting edge 602 abuts against the cover body 50, it means that the mouthpiece 60 is mounted in place. In the illustrated embodiment, the resisting edge 602 is formed by a shrinkage of a neck of the mouthpiece 60. It is understood that, in an alternative embodiment not shown, the resisting edge 602 can also be formed by extending outwardly from the circumferential surface of the mouthpiece 60 along a radial direction of the mouthpiece 60.

The liquid injection protection device further includes a second locking mechanism. The second locking mechanism includes a first connecting portion and a second connecting portion. It is understood that the configuration can be referred to the third embodiment. The first connecting portion is a latching groove arranged on the connection base 411. The second connecting portion is a latching member sliably arranged on the cover body 50 corresponding to the latching groove. After the locking member of the first locking mechanism is detached from the locking hole, it is further required to separate the latching member from the latching groove, then the liquid injection hole 402 can be opened by a rotation of the cover body 50.

In the atomizer 400 provided by the fourth embodiment of the present invention, when the user wants to perform a liquid injection, referring to FIG. 21, the mouthpiece 60 is required to be dismantled firstly, such that the locking relationship between the cover body 50 and the connection assembly 40 is removed. Then, referring to FIG. 22, the rotating member 44 is rotated, the cover plate is driven by the rotating member 44 to rotate, thereby opening the liquid injection hole 402. Because the liquid injection hole 402 can only be opened by a complicated operation procedure, thus the children cannot easily open the liquid injection hole 402, and a contact and ingestion of the e-cigarette liquid by the children can be avoided, it is therefore childproof. It is understood that, as the configuration in the first embodiment, a restricting recess 601 is provided in the mouthpiece 60 or in the cover body 50, the other one is provided with a restricting protrusion 5011, by an engagement between the restricting recess 601 and the restricting protrusion 5011, a range of movement of the mouthpiece 601 is restricted, therefore, the mouthpiece 60 is prevented from loss. When a liquid injection is required, the mouthpiece 60 is merely drawn upwards to enable the lower end of the mouthpiece 60 to be retracted into the through hole 501.

In the electronic cigarette provided by the fourth embodiment of the present invention, all technical features of the aforementioned atomizer 400 are included, thus the electronic cigarette has technical advantages the same as those in aforementioned atomizer 400.

### Fifth embodiment

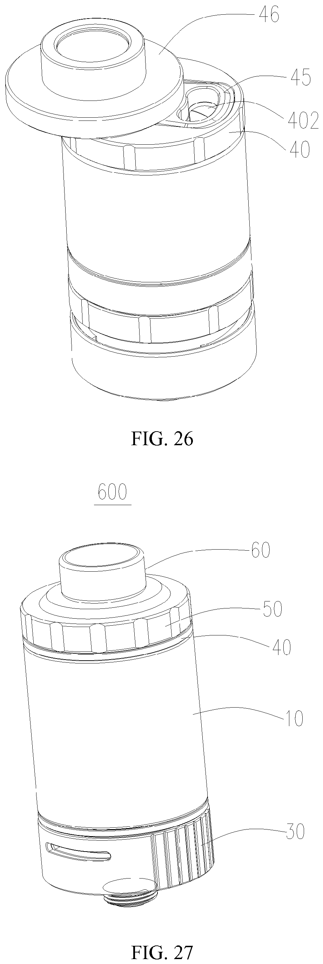

Referring to FIG. 23 and FIG. 26, an electronic cigarette is provided by the fifth embodiment of the present invention. The electronic cigarette includes an atomizer 500 and a battery device (not shown) electrically connected to the atomizer 500. Compared to the electronic cigarette of the third embodiment, the difference is that, the structure and the connecting mode of the connection assembly 40 and the cover body 50 in the atomizer 500 are different from the third embodiment, while the housing 10, the atomizing head 20 and the base assembly 30 are the same as the first embodiment, which is not specifically described herein.

Specifically, in the illustrated embodiment, the connection assembly 40 includes a connection base 411 connected to the upper end of the housing 10. The cover body 50 is arranged on the upper end of the connection base 411 and is slidable relative to the connection base 411. The mouthpiece 60 extends through the cover body 50 to be connected to the connection base 411.

The connection base 411 is inserted in the upper end of the housing 10. The connection base 411 has a substantially hollow cylindrical structure with an opening at a lower end. The connection base 411 is provided with a connecting post 4111 at the center of a bottom of the connection base 411 for the smoke to flow out. The upper end of the connecting post 4111 is provided with a liquid injection hole 402 at one side of the connecting post 4111. The liquid injection hole 402 is in communication with the liquid storage chamber 101, the user can inject the e-cigarette liquid into the liquid storage chamber 101 via the liquid injection hole 402.

In the illustrated embodiment, the cover body 50 includes a cover plate, the cover plate has a substantially hollow cylindrical structure with two openings at opposite ends. The inner chamber of the cover plate is in communication with the connecting post 4111. During the sliding process of the cover plate, the lower surface of the cover plate contacts the upper surface of the connection base 411, and the liquid injection hole 402 can be opened or closed.

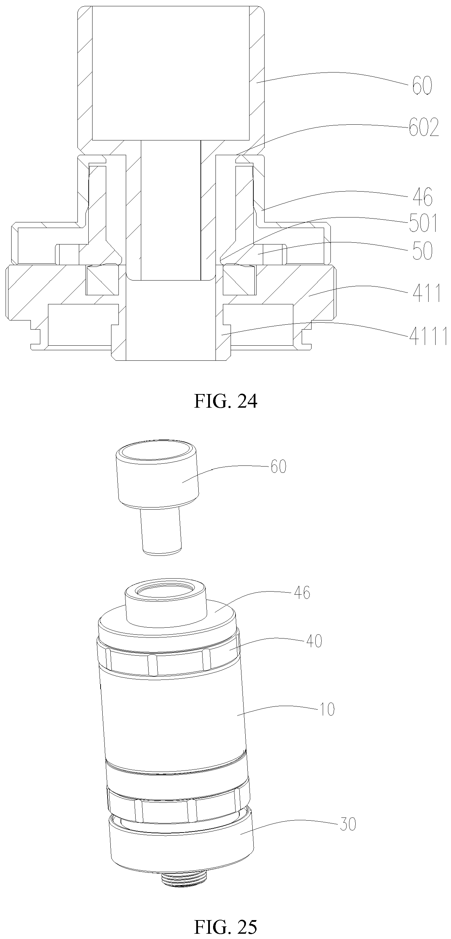

Referring to FIG. 24, in the illustrated embodiment, the cover body 50 further includes a sliding member 46 tightly sleeved on an outer side of the cover plate. Specifically, the sliding member 46 has a substantially cylindrical structure with a cross-section having a convex shape. When the sliding member 46 slides, the sliding member 46 drives the cover plate to slide together. The sliding member 46 provides a shielding function for the cover plate, enabling the atomizer 500 to have an attractive appearance.

In order to improve a tightness of the liquid injection hole 402 sealed by the cover body 50, the connection base 411 is provided with a sealing pad 45 surrounding a periphery of the liquid injection hole 402, for avoiding an e-cigarette liquid leakage.

In the illustrated embodiment, the upper surface of the connection base 411 is provided with a sliding rail (not shown), the lower surface of the cover plate is provided with a sliding block (not shown) matching the sliding rail. When the sliding member 46 drives the cover plate to slide, the sliding block can slide along the sliding rail, ensuring a sliding stability of the cover body 50. It is understood that, in an alternative embodiment not shown, the sliding rail is arranged on the cover plate, the sliding block is arranged on the connection base 411 and engages with the sliding rail, to provide a guiding for the sliding of the cover body 50.

It is understood that, in an alternative embodiment not shown, the sliding member 46 can also be omitted, the user can open or close the liquid injection hole 402 by sliding the cover plate.

Further, the atomizer 500 further includes a first locking mechanism. It is understood that, the connection assembly 40, the cover body 50 and the first locking mechanism cooperatively serve as a liquid injection protection device. The first locking mechanism includes a locking hole provided in the cover body 50 and a locking member extending through the locking hole to be detachably connected to the connection assembly 40. In the illustrated embodiment, the locking hole is the through hole 501 provided in the cover body 50, the locking member is the mouthpiece 60 extending through the through hole 501 to be connected to the connection assembly 40.

The mouthpiece 60 has a substantially hollow cylindrical structure with two openings at opposite ends. After the mouthpiece 60 extends through the through hole 501 of the cover body 50, the mouthpiece 60 is tightly inserted into the connection base 411. At the same time, the liquid injection hole 402 is sealed by the cover body 50, and the mouthpiece 60 is in communication with the smoke outlet passage 423, the user can inhale by sucking the mouthpiece 60 with mouth.

In the illustrated embodiment, when the mouthpiece 60 is connected to the connection assembly 40, a sliding of the cover body 50 is restricted by the mouthpiece 60. The mouthpiece 60 has an outer diameter equal to an inner diameter of the though hole 501 of the cover body 50. Specifically, the outer diameter of the portion of the mouthpiece 60 inserted in the through hole 501 is equal to the inner diameter of the through hole 501. Since there is no movable space between the mouthpiece 60 and the through hole 501, the cover body 50 cannot be flipped relative to the mouthpiece 60, and the cover body 50 is locked. When the mouthpiece 60 is dismantled, the cover body 50 is then rotatable.

Further, the external wall of the mouthpiece 60 is provided with a resisting edge 602. When the mouthpiece 60 is connected to the connection assembly 40, the resisting edge 602 abuts against the cover body 50, thereby providing a restricting function for the mounting of the mouthpiece 60, That is, when the resisting edge 602 abuts against the cover body 50, it means that the mouthpiece 60 is mounted in place. In the illustrated embodiment, the resisting edge 602 is formed by a shrinkage of a neck of the mouthpiece 60. It is understood that, in an alternative embodiment not shown, the resisting edge 602 can also be formed by extending outwardly from the circumferential surface of the mouthpiece 60 along a radial direction of the mouthpiece 60.

The liquid injection protection device further includes a second locking mechanism. The second locking mechanism includes a first connecting portion and a second connecting portion. It is understood that the configuration can be referred to the third embodiment. The first connecting portion is a latching groove arranged on the connection base 411. The second connecting portion is a latching member sliably arranged on the cover body 50 corresponding to the latching groove. After the locking member of the first locking mechanism is detached from the locking hole, it is further required to separate the latching member from the latching groove, then the liquid injection hole 402 can be opened by a sliding of the cover body 50.

In the atomizer 500 provided by the fifth embodiment of the present invention, when the user wants to perform a liquid injection, referring to FIG. 25, the mouthpiece 60 is required to be dismantled firstly, such that the locking relationship between the cover body 50 and the connection base 411 of the connection assembly 40 is removed. Then, referring to FIG. 26, the sliding member 46 is caused to slide, the cover plate is driven to slide together, thereby opening the liquid injection hole 402. Because the liquid injection hole 402 can only be opened by a complicated operation procedure, thus the children cannot open the liquid injection hole casually, thereby a contact and ingestion of the e-cigarette liquid by the children can be avoided, it is therefore childproof. It is understood that, as the configuration in the first embodiment, a restricting recess 601 is provided in the mouthpiece 60 or in the cover body 50, the other one is provided with a restricting protrusion 5011, by an engagement between the restricting recess 601 and the restricting protrusion 5011, a range of movement of the mouthpiece 601 is restricted, therefore, the mouthpiece 60 is prevented from loss. When a liquid injection is required, the mouthpiece 60 is merely drawn upwards to enable the lower end of the mouthpiece 60 to be retracted into the through hole 501.

In the electronic cigarette provided by the fifth embodiment of the present invention, all technical features of the aforementioned atomizer 500 are included, thus the electronic cigarette has technical advantages the same as those in aforementioned atomizer 500.

### Sixth embodiment

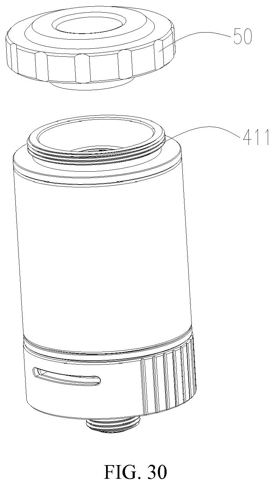

Referring to FIG. 27 and FIG. 30, an electronic cigarette is provided by the sixth embodiment of the present invention. The electronic cigarette includes an atomizer 600 and a battery device (not shown) electrically connected to the atomizer 600. Compared to the electronic cigarette of the third embodiment, the difference is that, the structure and the connecting mode of the connection assembly 40 and the cover body 50 in the atomizer 600 are different from the third embodiment, while the housing 10, the atomizing head 20 and the base assembly 30 are the same as the first embodiment, which is not specifically described herein.

In the illustrated embodiment, the connection assembly 40 includes a connection base 411 connected to an upper end of the housing 10 and a ventilation member 42 connected to the lower end of the connection base 411. The cover body 50 is detachably connected to the connection base 411. The mouthpiece 60 extends through the cover body 50 to be connected to the ventilation member 42.

The connection base 411 is inserted in the upper end of the housing 10. The connection base 411 has a substantially hollow cylindrical structure with opening at an upper end. The connection base 411 is provided with a connecting post 4111 at the center of a bottom of the connection base 411 for the smoke to flow out. The lower end of the connecting post 4111 is provided with two liquid injection holes 402 at opposite sides of the connecting post 4111. The liquid injection holes 402 are in communication with the liquid storage chamber 101, the user can inject the e-cigarette liquid into the liquid storage chamber 101 via the liquid injection hole 402. It is understood that, the number of the liquid injection holes 402 can be one or more than two.

The cover body 50 has a substantially hollow cylindrical structure with an opening at a lower end. The cover body 50 covers the connection base 411 and is detachably connected to the connection base 411 via threads. When the cover body 50 covers the connection base 411, the cover body 50 can seal the liquid injection hole 402. In the illustrated embodiment, the cover body 50 is provided with a through hole 501 at a center thereof. It is understood that, in an alternative embodiment not shown, the cover body 50 can be detachably connected to the connection base 411 by latching or plugging.

The ventilation member 42 has a substantially hollow cylindrical structure with two openings at opposite ends. The external wall of the upper end of the ventilation member 42 is connected to the internal wall of the connecting post 4111 by an interference fit. The lower end of the ventilation member 42 is connected to the atomizing head 20 via threads. The interior chamber of the ventilation member 42 forms a smoke outlet passage 423. When in use, the smoke generated in the atomizer 20 enters into the user's mouth via the smoke outlet passage 423. It is understood that, in an alternative embodiment not shown, the upper end of the ventilation member 42 can be connected to the connecting post 4111 via a threaded connection or latching, the lower end of the ventilation member 42 can be connected to the atomizing head 20 via a threaded connection or latching.

It is understood that, in an alternative embodiment not shown, the upper end of the ventilation member 42 is tightly sleeved on an outer side of the connecting post 4111, thus a connection between the ventilation member 42 and the connecting post 4111 can also be established.

The atomizer 600 further includes a first locking mechanism. It is understood that, the connection assembly 40, the cover body 50 and the first locking mechanism cooperatively serve as a liquid injection protection device. The first locking mechanism includes a locking hole provided in the cover body 50 and a locking member extending through the locking hole to be detachably connected to the connection assembly 40. In the illustrated embodiment, the locking hole is the through hole 501 provided in the cover body 50, the locking member is the mouthpiece 60 extending through the through hole 501 to be connected to the connection assembly 40.

In the illustrated embodiment, the mouthpiece 60 has a substantially hollow cylindrical structure with two openings at opposite ends. After one end of the mouthpiece 60 extends through the cover body 50, the mouthpiece 60 is tightly inserted into the upper end of the ventilation member 42, and the mouthpiece 60 is in communication with the smoke outlet passage 423, the user can inhale by sucking the mouthpiece 60 with mouth. Specifically, after the lower end of the mouthpiece 60 extends through the through hole 501 of the cover body 50, the lower end of the mouthpiece 60 is connected to the ventilation member 42 by a threaded connection. It is understood that, when the upper end of the ventilation member 42 is tightly sleeved on the outer of the connecting post 4111, the lower end of the mouthpiece 60 is connected to the connecting post 4111 via a threaded connection. It can also be understood that, in an alternative embodiment not shown, the lower end of the mouthpiece 60 can be detachably connected to the ventilation member 42 or the connecting member 4111 by latching or plugging.

Further, the external wall of the mouthpiece 60 is provided with a resisting edge 602. When the cover body 50 seals the liquid injection hole 402, the resisting edge 602 abuts against the cover body 50, thereby an axial movement of the cover body 50 is restricted. That is, the cover body 50 is locked, and a detachment of the cover body 50 from the connection base 411 is avoided. In the illustrated embodiment, the resisting edge 602 is formed by a shrinkage of a neck of the mouthpiece 60. It is understood that, in an alternative embodiment not shown, the resisting edge 602 can also be formed by extending outwardly from the circumferential surface of the mouthpiece 60 along a radial direction of the mouthpiece 60. In addition, the resisting edge 602 provides a restricting function for the mounting of the mouthpiece 60. When the resisting edge 602 abuts against the cover body 50, it means that the mouthpiece 60 is mounted in place.

The liquid injection protection device further includes a second locking mechanism. The second locking mechanism includes a first connecting portion and a second connecting portion. It is understood that the configuration can be referred to the third embodiment. The first connecting portion is a latching groove arranged on the connection base 411. The second connecting portion is a latching member sliably arranged on the cover body 50 corresponding to the latching groove. After the locking member of the first locking mechanism is detached from the locking hole, it is further required to separate the latching member from the latching groove, then the liquid injection hole 402 can be opened by unscrewing the cover body 50.

In the atomizer 600 provided by the sixth embodiment of the present invention, when the user wants to perform a liquid injection, the mouthpiece 60 is required to be dismantled firstly, such that the locking relationship between the cover body 50 and the connection base 411 of the connection assembly 40 is removed. Then, the cover body 50 is disassembled from the connection base 411, the liquid injection hole 402 is thereby opened. Therefore, the liquid injection hole 402 can only be opened by a complicated operation procedure, thus the children cannot open the liquid injection hole casually, and a contact and ingestion of the e-cigarette liquid by the children can be avoided, it is therefore childproof. It is understood that, as the configuration in the first embodiment, a restricting recess 601 is provided in the mouthpiece 60 or in the cover body 50, the other one is provided with a restricting protrusion 5011, by an engagement between the restricting recess 601 and the restricting protrusion 5011, a range of movement of the mouthpiece 601 is restricted, therefore, the mouthpiece 60 is prevented from loss. When a liquid injection is required, the mouthpiece 60 is merely drawn upwards to enable the lower end of the mouthpiece 60 to be retracted into the through hole 501.

In the electronic cigarette provided by the sixth embodiment of the present invention, all technical features of the aforementioned atomizer 600 are included, thus the electronic cigarette has technical advantages the same as those in aforementioned atomizer 600.

It is understood that, the configuration of the liquid injection protection device in the second embodiment to the sixth embodiment can refer to the first embodiment, a movable ring is added, the movable ring is movably sleeved on the connection assembly 40 to avoid a dismantlement of the connection assembly 40.

The embodiments described above are merely preferred embodiments, but not intended to limit the application. Any modifications, alternatives or improvements made within the principle of the present invention should be interpreted as falling within the protection scope of the present invention. The claims are not limited to the features or acts described above. Rather, the proper scope of the present invention is defined by the appended claims.

## Claims

1. A liquid injection protection device comprising a cover body, a connection assembly and a first locking mechanism, wherein the connection assembly is provided with a liquid injection hole, the cover body covers the liquid injection hole and is movable relative to the connection assembly, the first locking mechanism comprises a locking hole provided in the cover body and a locking member capable of extending through the locking hole to be detachably connected to the connection assembly, when one end of the locking member extends through the locking hole to be connected to the connection assembly, a movement of the cover body is restricted by the locking member, so as to prevent the movement of the cover body to open the liquid injection hole.

2. The liquid injection protection device of claim 1, wherein an outer diameter of the portion of the locking member inserted in the locking hole is equal to an inner diameter of the locking hole, and/or an outer wall of the locking member is provided with a resisting edge, when the one end of the locking member extends through the locking hole to be connected to the connection assembly, the cover body is restricted between the resisting edge and the connection assembly.

3. The liquid injection protection device of claim 1, wherein a sidewall of the locking hole is provided with a restricting protrusion, an external wall of the locking member is provided with a restricting recess, or, the sidewall of the locking hole is provided with a restricting recess, the external wall of the locking member is provided with a restricting protrusion, the restricting protrusion is movably embedded in the restricting recess for preventing a detachment of the locking member from the cover body.

4. The liquid injection protection device of claim 1, further comprising a movable ring, wherein the movable ring is rotatably sleeved on an outer side of the connection assembly for preventing the connection assembly from being dismantled.

5. The liquid injection protection device of claim 1, wherein the locking member is a mouthpiece.

6. The liquid injection protection device of any one of claims 1 to 5, wherein the connection assembly comprises a connection base, the cover body is detachably connected to the connection base, or, the cover body is movably arranged on the connection base.

7. The liquid injection protection device of claim 6, wherein one end of the cover body is hinged to the connection base, allowing the cover body to be flipped relative to the connection base.

8. The liquid injection protection device of claim 7, wherein a sealing member is protruded from a lower surface of the cover body corresponding to the liquid injection hole, when the cover body covers the connection base, the sealing member is latched into the liquid injection hole.

9. The liquid injection protection device of claim 6, wherein the cover body is rotatably arranged on the connection base, a rotation center of the cover body is staggered from a central axis of the connection base.

10. The liquid injection protection device of claim 6, wherein the cover body is slidably arranged on the connection base, the connection base is provided with a sliding rail, and the cover body is provided with a sliding block matching the sliding rail, or, the connection base is provided with a sliding block, and the cover body is provided with a sliding rail matching the sliding block.

11. The liquid injection protection device of any one of claims 1 to 5, further comprising a second locking mechanism, wherein the second locking mechanism comprises a first connecting portion arranged on the connection assembly and a second connecting portion arranged on the cover body, after a connection between the locking member and the connection assembly is removed, the cover body is further required to be moved to remove an engagement between the first connecting portion and the second connecting portion so as to separate the first connecting portion from the second connecting portion, and then the liquid injection hole is able to be opened.

12. The liquid injection protection device of claim 11, wherein the connection assembly is provided with a sliding groove, the cover body is provided with a rotating shaft, when the cover body is moved, the rotating shaft slides along the sliding groove, when the cover body is moved to a position permitting the first connecting portion to separate from the second connecting portion, the cover body is capable of rotating around the rotating shaft relative to the connection assembly to open the liquid injection hole.

13. The liquid injection protection device of claim 12, wherein the connection assembly comprises a connection base and a gland covering the connection base, the liquid injection hole is provided in the connection base, the gland is provided with a cutout with an opening at one end, the cutout is formed by recessing from one side edge of the gland, the liquid injection hole is received in and exposed from the cutout.

14. The liquid injection protection device of claim 13, wherein the cutout comprises a connecting wall located at a side of the liquid injection hole and two sidewalls respectively connected to opposite ends of the connecting wall and extending in the same direction, the surface of the connecting wall adjacent to the connection base is provided with a recess, the recess is recessed from the connecting wall along a direction away from the cutout, the sliding groove is provided at one end of each sidewall connecting with the connecting wall, the sliding groove is recessed from the surface of the sidewall adjacent to the connection base, the sliding grooves provided at the two surfaces of the two sidewalls are disposed oppositely to each other, the sliding grooves are in communication with the cutout and the recess.

15. The liquid injection protection device of claim 14, wherein the cover body comprises a movable member, the rotating shaft is arranged on opposite sides of one end of the movable member, a covering portion is formed by extending outwardly from opposite sides of the other end of the movable member opposite to the rotating shaft, the gland is provided with a locking groove at a bottom of one end of the gland away from the connecting wall, the locking groove is in communication with the cutout, the covering portion serves as the second connecting portion, the locking groove serves as the first connecting portion, when the movable member is moved to cause the covering portion to engage into the locking groove, one end of the movable member adjacent to the rotating shaft is restricted in the recess, when the movable member is moved to cause the covering portion to disengage from the locking groove, the one end of the movable member adjacent to the rotating shaft is moved out of the recess and the movable member is able to be flipped.

16. The liquid injection protection device of claim 12, wherein the connection assembly comprises a lining member and a stopping member, the liquid injection hole is provided in the lining member, the lining member is further provided with an embedding groove, the stopping member is embedded in the embedding groove, the sliding groove is provided at one end of the stopping member along a length direction of the stopping member, the cover body comprises a clamping member, the rotating shaft is arranged on one end of the clamping member.

17. The liquid injection protection device of claim 16, wherein the connection assembly further comprises a snapping member, the snapping member is arranged in the embedding groove and located under one end of the stopping member away from the sliding groove, the one end of the stopping member away from the sliding groove is provided with an insertion opening, the snapping member and the insertion opening cooperatively serve as the first connecting portion, the clamping member is provided with a claw at one end away from the rotating shaft, the claw serves as the second connecting portion, when the clamping member is moved to cause the claw to separate from the snapping member, the claw is aligned with the insertion opening, and the clamping member is able to be flipped.

18. The liquid injection protection device of claim 17, wherein the claw is provided with a latching groove, the snapping member is provided with a protrusion corresponding to the latching groove, when the first connecting portion is engaged with the second connecting portion, the protrusion latches into the latching groove, or, the snapping member and the claw are each provided with a magnet member, when the first connecting portion is engaged with the second connecting portion, the snapping member and the claw are attracted by the magnet members.

19. The liquid injection protection device of claim 16, wherein the connection assembly further comprises an elastic member, when the clamping member is moved, the elastic member always resists one end of the clamping member having the rotating shaft.

20. An atomizer comprising the liquid injection protection device of any one of claims 1 to 19.

21. The atomizer of claim 20, further comprising a housing, a base assembly and an atomizing head, wherein the connection assembly is arranged at one end of the housing, the base assembly is arranged at the other end of the housing opposite to the connection assembly, a liquid storage chamber in communication with the liquid injection hole is provided in the housing, the atomizing head is received in the liquid storage chamber.

22. An electronic cigarette comprising the atomizer of claim 20 or 21.
